(19) Europäisches Patentamt
European Patent Office
Office européen des brevets

(11) Veröffentlichungsnummer: **0 399 196**
**A1**

(12) **EUROPÄISCHE PATENTANMELDUNG**

(21) Anmeldenummer: **90106877.5**

(22) Anmeldetag: **10.04.90**

(51) Int. Cl.[5]: **C07D 417/12, C07D 417/14, C07C 403/12, A61K 31/54, A61K 31/53, //(C07D417/12, 285:00,233:00),(C07D417/14, 285:00,233:00,211:00), (C07D403/12,253:00,233:00)**

(30) Priorität: **25.04.89 DE 3913597**

(43) Veröffentlichungstag der Anmeldung:
**28.11.90 Patentblatt 90/48**

(84) Benannte Vertragsstaaten:
**AT BE CH DE DK ES FR GB GR IT LI LU NL SE**

(71) Anmelder: **HEUMANN PHARMA GMBH & CO**
**Heideloffstrasse 18 - 28**
**D-8500 Nürnberg 1(DE)**

(72) Erfinder: **Mörsdorf, Peter Dr., Dipl.-Chem.**
**Ziegelstrasse 64**
**D-8506 Langenzenn(DE)**
Erfinder: **Engler, Heidrun Dr.**
**Ringstrasse 23**
**D-8501 Cadolzburg(DE)**
Erfinder: **Weidner, Reinhold Dr., Dipl.-Chem.**
**Friedenstrasse 15**
**D-8500 Nürnberg(DE)**
Erfinder: **Herter, Rolf Dr., Dipl.-Chem.**
**Kreuzweg 12**
**D-8411 Zeitlarn(DE)**
Erfinder: **Ahrens, Kurt-Henning Dr.**
**Trödelmarkt 42**
**D-8500 Nürnberg(DE)**

(74) Vertreter: **Kraus, Walter, Dr. et al**
**Patentanwälte Kraus, Weisert & Partner**
**Thomas-Wimmer-Ring 15**
**D-8000 München 22(DE)**

(54) **Diazinderivate, Verfahren zu ihrer Herstellung und diese Verbindungen enthaltende Arzneimittel.**

(57) Beschrieben werden neue Diazinderivate der allgemeinen Formel I

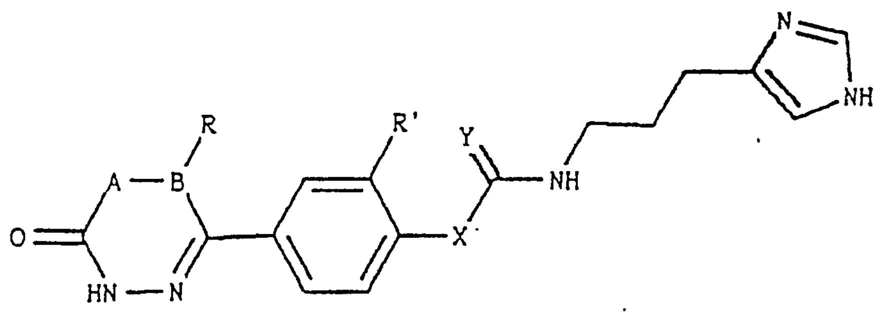

(I)

sowie die physiologisch annehmbaren Salze davon. Die erfindungsgemäßen Verbindungen stellen neue positiv inotrope Verbindungen mit höherer und/oder selektiverer Wirkung als derzeit bekannte Verbindungen dar und sind daher zur Behandlung und Vorbeugung von Erkrankungen des Herzens und des Kreislaufs geeignet.

EP 0 399 196 A1

Xerox Copy Centre

## Diazinderivate, Verfahren zu ihrer Herstellung und diese Verbindungen enthaltende Arzneimittel

Digitalisglykoside, wie Digoxin und Digitoxin, und Sympathomimetika waren für viele Jahrzehnte in der Behandlung der Herzinsuffizienz die einzigen Therapiemöglichkeiten. Die ausgeprägten Nachteile beider Substanzgruppen, wie geringe therapeutische Breite, Tachyphylaxie und mangelhafte orale Verfügbarkeit führten zu einer intensiven Suche nach weiteren positiv inotrop wirksamen Substanzklassen.

Pyridazinonderivate, wie Pimobendan (DE-OS-28 37 161), Imazodan (EP-OS-0 75 436) und Indolidan (US-PS-4 591 591) erwiesen sich dabei aufgrund ihrer guten kontraktilitätssteigernden Wirkung als eine Alternative. Ebenso wurden bioisostere Verbindungsgruppen, z.B. Thiadiazinone und Triazinone (EP-OS-0 52 442) mit ähnlichen pharmakologischen Eigenschaften beschrieben.

Histamin-$H_2$-Agonisten andererseits, wie z.B. Impromidin (J. Med. Chem. 28, 1414 (1985)), sind eine weiter interessante Gruppe von cardiotonen Substanzen, deren Wirkung auf einem von dem der Pyridazinonderivate verschiedenen Mechanismus beruht.

Der Erfindung liegt daher die Aufgabe zugrunde, neue positiv inotrope Verbindungen mit höherer und/oder selektiverer Wirksamkeit als die derzeit bekannten Verbindungen zur Verfügung zu stellen. Diese Aufgabe wird durch die Erfindung gelöst.

Gegenstand der Erfindung sind daher Diazinderivate der allgemeinen Formel I

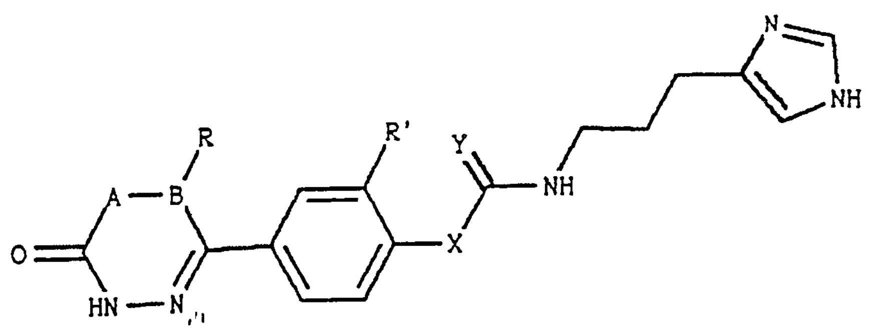

(I)

in der A für ein Schwefelatom und B für ein Kohlenstoffatom steht oder eines der beiden Atome A und B ein unsubstituiertes oder mit einer $C_1$-$C_3$-Alkylgruppe substituiertes Stickstoffatom und das andere ein Kohlenstoffatom bedeuten, R ein Wasserstoffatom, eine $C_1$-$C_3$-Alkylgruppe oder eine Hydroxymethylgruppe und R′ ein Wasserstoffatom, eine Nitrogruppe, eine Cyanogruppe oder ein Halogenatom bedeutet, X für eine der Gruppen

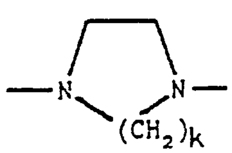

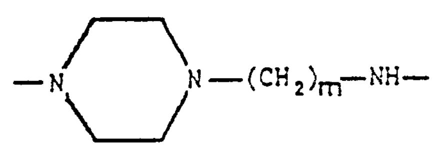

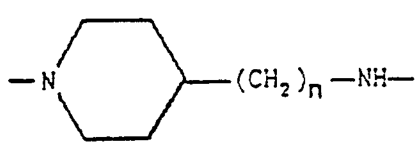

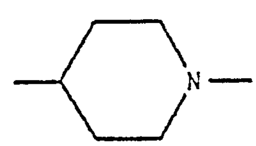

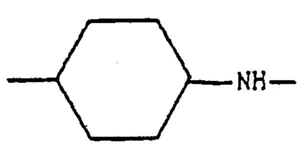

$-NH(CH_2)_m-NH-$, $-O(CH_2)_n-NH-$ und $-(CH_2)_n-NH-$ steht, worin k den Wert 2 oder 3, m den Wert 2,3,4,5 oder

6 und n den Wert 0,1,2,3 oder 4 haben, und Y für ein Sauerstoffatom oder eine der Gruppen

$=NH$ $=N-C(=O)-R^1$ und $=N-C(=O)-OR^2$

steht, worin $R^1$ für eine gerad- oder verzweigtkettige $C_1$-$C_6$-Alkylgruppe oder eine gegebenenfalls mit einem oder mehreren Halogenatomen, $C_1$-$C_3$-Alkylgruppen oder $C_1$-$C_3$-Alkoxygruppen. substituierte Arylgruppe steht und $R^2$ eine gegebenenfalls mit einem oder mehreren Halogenatomen, $C_1$-$C_3$-Alkoxygruppen oder Phenylresten substituierte, gerad-oder verzweigtkettige $C_1$-$C_4$-Alkylgruppe bedeutet, sowie die physiologisch annehmbaren Salze davon.

In der allgemeinen Formel I bedeutet A ein Schwefelatom und B ein Kohlenstoffatom oder eines der beiden Atome A und B steht für ein unsubstituiertes oder mit einer $C_1$-$C_3$-Alkylgruppe substituiertes Stickstoffatom und das andere für ein Kohlenstoffatom. Beispiele für die $C_1$-$C_3$-Alkylgruppen sind dabei die Methyl-, Ethyl oder n-Propylgruppe, wobei die Methylgruppe bevorzugt wird.

R steht für ein Wasserstoffatom, eine $C_1$-$C_3$-Alkylgruppe oder eine Hydroxymethylgruppe. Die $C_1$-$C_3$-Alkylgruppe ist wie oben definiert, wobei die Methylgruppe wiederum bevorzugt wird. $R'$ bedeutet eine Nitrogruppe, eine Cyanogruppe oder ein Halogenatom, beispielsweise ein Fluor-, Chlor- oder Bromatom, vorzugsweise ein Fluoratom. Besonders bevorzugt sind jedoch Verbindungen, bei denen $R'$ für eine Nitrogruppe oder eine Cyanogruppe steht.

X steht für die Gruppen

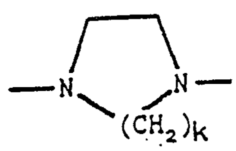

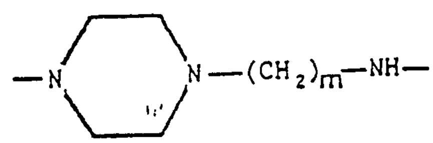

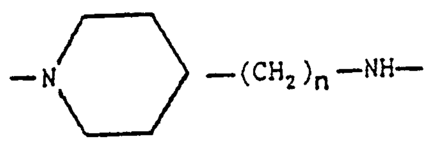

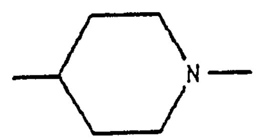

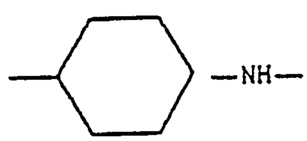

$-NH(CH_2)_mNH-$, $-O(CH_2)_nNH-$ oder $-(CH_2)_nNH-$. Der Index k hat dabei den Wert 2 oder 3, wobei der Wert 2 bevorzugt wird. Der Index m hat den Wert 2,3,4,5 oder 6, wobei die Werte 2,3 und 4 bevorzugt werden; n steht für eine ganze Zahl von 0 bis 4. Y steht für ein Sauerstoffatom oder die Gruppen

$=NH$, $=N-C(=O)-R^1$ oder $=N-C(=O)-OR^2$

Dabei steht $R^1$ für eine gerad- oder verzweigtkettige $C_1$-$C_6$-Alkylgruppe, vorzugsweise eine $C_1$-$C_3$-Alkylgruppe, oder eine gegebenenfalls mit Halogenatomen, $C_1$-$C_3$-Alkylgruppen oder $C_1$-$C_3$-Alkoxygruppen ein- bis dreifach substituierte Arylgruppe, wobei die Einfachsubstitution, insbesondere in para-Stellung,bevorzugt wird.

Beispiele für die $C_1$-$C_6$-Alkylgruppe sind die Methyl-, Ethyl-, n-Propyl-, i-Propyl-, n-Butyl- und i-Butylgruppe. Die Arylgruppe kann beispielsweise eine Phenyl-oder Naphthylgruppe sein, wobei die Phenylgruppe bevorzugt wird. Beispiele für Halogenatome und $C_1$-$C_3$-Alkylgruppen,die Substituenten der Arylgruppe bilden, sind die oben im Zusammenhang mit den Gruppen R und $R'$ aufgeführten Gruppen. Beispiele für die $C_1$-$C_3$-Alkoxygruppen, die ebenfalls Substituenten der Arylgruppe bilden, sind die Methoxy-, Ethoxy- und n-Propoxygruppe.

$R^2$ ist eine gerad- oder verzweigtkettige $C_1$-$C_4$-Alkylgruppe, die unsubstituiert sein kann oder mit ein oder mehreren Halogenatomen, $C_1$-$C_3$-Alkoxygruppen oder Phenylresten substituiert sein kann. Beispiele für die $C_1$-$C_4$-Alkylgruppe sind die Methyl-, Ethyl-, n-Propyl-, i-Propyl-, n-Butyl, i-Butyl und tert-Butylgruppe, wobei die Methyl-, Ethyl- oder tert-Butylgruppe bevorzugt wird. Im Falle einer Substitution mit den

genannten Substituenten wird die Einfachsubstitution, insbesondere am endständigen C-Atom,bevorzugt. Beispiele für Halogenatome, die die Substituenten der $C_1$-$C_4$-Alkylgruppe bilden, sind wiederum die Fluor-, Chlor- oder Bromatome, wobei Fluor- oder Chloratome bevorzugt werden. Beispiele für $C_1$-$C_3$-Alkoxygruppen, die ebenfalls Substituenten der durch $R^2$ angegebenen $C_1$-$C_4$-Alkylgruppe bilden können, sind die Methoxy-, Ethoxy-und n-Propoxygruppe, wobei die Methoxy- oder Ethoxygruppe bevorzugt wird.

Bevorzugt hat Y die Bedeutung

$=NH$ oder $=N-\underset{\underset{O}{\|}}{C}-OR^2$

wobei die Bedeutung $=NH$ besonders bevorzugt wird, wobei $R^2$ wie oben definiert ist und vorzugsweise für eine Ethyl-oder tert.-Butylgruppe steht.

Eine bevorzugte Gruppe von erfindungsgemäßen Verbindungen ist dadurch gekennzeichnet, daß A für ein Schwefelatom und B für ein Kohlenstoffatom stehen, R ein Wasserstoffatom, eine $C_1$-$C_3$-Alkylgruppe oder eine Hydroxymethylgruppe und R' ein Wasserstoffatom, eine Nitrogruppe, eine Cyanogruppe oder ein Halogenatom bedeutet, X für eine der Gruppen

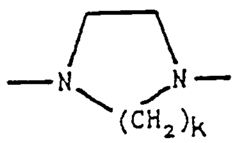

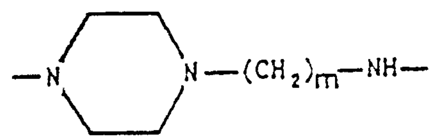

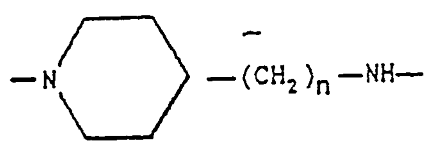

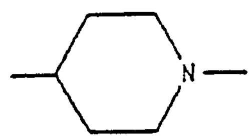

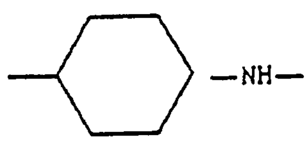

$-NH(CH_2)_m-NH-$, $-O(CH_2)_n-NH-$ und $-(CH_2)_n-NH-$ steht, worin k den Wert 2 oder 3, m den Wert 2,3,4,5 oder 6 und n den Wert 0,1,2,3 oder 4 haben und Y für die Gruppe $=NH$ steht.

Eine weitere bevorzugte Gruppe von erfindungsgemäßen Verbindungen ist dadurch gekennzeichnet, daß A für ein Stickstoffatom oder die Gruppe $-N-CH_3$ und B für ein Kohlenstoffatom stehen, R ein Wasserstoffatom oder eine $C_1$-$C_3$-Alkylgruppe und R' ein Wasserstoffatom, eine Nitrogruppe, eine Cyanogruppe oder ein Halogenatom bedeutet und Y für die Gruppe $=NH$ steht. Verbindungen dieser Gruppe, bei denen R eine Methylgruppe ist und R' eine Nitrogruppe ist, werden besonders bevorzugt.

Eine weitere bevorzugte Gruppe von erfindungsgemäßen Verbindungen ist dadurch gekennzeichnet, daß A für ein Kohlenstoffatom und B für ein Stickstoffatom steht, R ein Wasserstoffatom oder eine Methylgruppe ist und R' ein Wasserstoffatom, eine Nitrogruppe, eine Cyanogruppe oder ein Halogenatom bedeutet und Y für die Gruppe $=NH$ steht.

Die nachstehend aufgeführten Einzelverbindungen werden besonders bevorzugt.

5-[4-[4-[3-(1H-Imidazol-4-yl)propylamino-iminomethylen]-piperazin-1-yl]-3-nitrophenyl]-6-methyl-3H.6H-1,3,4-thiadiazin-2-on sowie die physiologisch annehmbaren Salze davon.

N'-[3-(1H-Imidazol-4-yl)propyl]-$N^3$-[1-[4-(6-methyl-2-oxo-3,6-dihydro-1,3,4-thiadiazin-5-yl)-2-nitro-phenyl]-piperidin-4-yl]-guanidin sowie die physiologisch annehmbaren Salze davon.

5-[4-[4-[3-(1H-Imidazol-4-yl)propylamino-iminomethylen]-piperazin-1-yl]-3-nitro-phenyl]-3H.6H-1,3,4-thiadiazin-2-on sowie die physiologisch annehmbaren Salze davon.

N'-[3-(1H-Imidazol-4-yl)propyl]-$N^3$-[1-[4-(2-oxo-3,6-dihydro-1,3,4-thiadiazin-5-yl)-2-nitro-phenyl]-piperidin-4-yl]-guanidin sowie die physiologisch annehmbaren Salze davon.

Die erfindungsgemäßen Verbindungen können auf folgende Weise hergestellt werden:

Verbindungen der allgemeinen Formel I, bei denen A, B, R, R' und X die obengenannten Bedeutungen besitzen und Y für eine der Gruppen

$=NH$ $=N-\underset{\underset{O}{\|}}{C}-R'$ und $=N-\underset{\underset{O}{\|}}{C}-OR^2$

steht, worin $R^1$ und $R^2$ wie oben definiert sind, können dadurch hergestellt werden, daß man eine Verbindung der allgemeinen Formel II

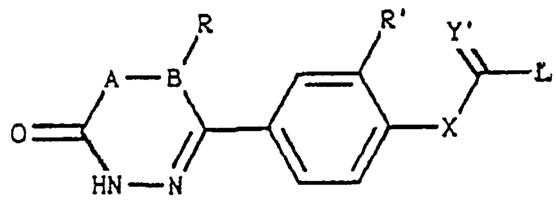

(II)

in der A, B, R, R′, X und Y wie oben definiert sind und L für eine $C_1$-$C_4$-Alkylthio-, eine Phenylthio-, eine $C_1$-$C_4$-Alkoxy- oder eine Phenoxygruppe steht, mit einer Verbindung der Formel III

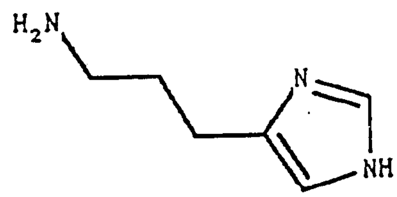

(III)

zu einer Verbindung der allgemeinen Formel I umsetzt.

Die obige Verbindungsgruppe kann auch dadurch hergestellt werden, daß man eine Verbindung der allgemeinen Formel IV

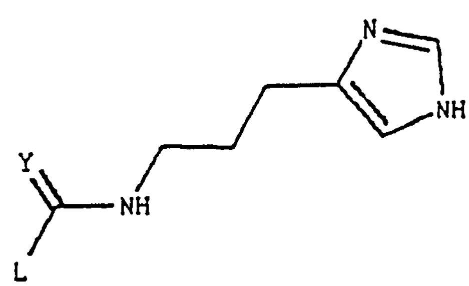

(IV)

in der Y und L die oben im Zusammenhang mit dem ersten Verfahren angegebenen Bedeutungen besitzen, mit einer Verbindung der allgemeinen Formel V

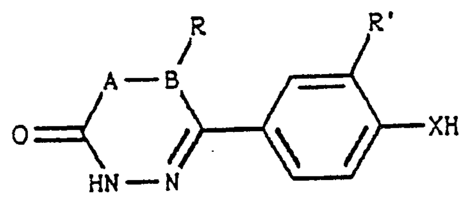

(V)

in der A, B, R, R′ und X wie oben definiert sind, umgesetzt werden.

Bei beiden Verfahrensvarianten werden als Austrittsgruppen L in den Verbindungen der Formeln II und IV eine $C_1$-$C_4$-Alkylthiogruppe, insbesondere die Methylthiogruppe, und die Phenoxygruppe bevorzugt. Die Verbindungen der allgemeinen Formeln II und IV werden vorzugsweise im Verhältnis 1:1 bis 0,8:1, besonders bevorzugt in äquimolaren Mengen, bezogen auf die Verbindungen der allgemeinen Formeln III bzw. V, eingesetzt. Die Umsetzungen werden in einem polaren Lösungsmittel, wie Acetonitril, Pyridin,

Dimethylformamid, oder einem Alkohol, vorzugsweise einem sekundären oder tertiären Alkohol, wie zum Beispiel Isopropanol, und bei Temperaturen von 20 °C bis zur Rückflußtemperatur des verwendeten Lösungsmittels durchgeführt.

Verbindungen der allgemeinen Formel I, bei denen A, B, R, R′ und X wie oben definiert sind und Y für die Gruppe =NH steht, können auch dadurch hergestellt werden, daß man eine Verbindung der allgemeinen Formel Ia

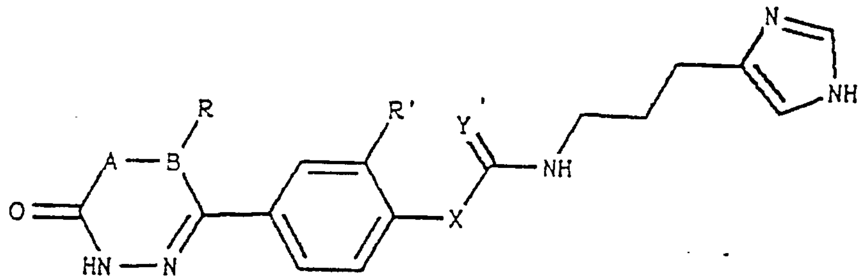

(Ia)

in der A, B, R, R′ und X die oben angegebenen Bedeutungen haben und Y′ für eine der Gruppen
$=N-\underset{\underset{O}{\|}}{C}-R'$ oder $=N-\underset{\underset{O}{\|}}{C}-OR^2$
steht, wobei R' und $R^2$ die oben angegebenen Bedeutungen haben, sauer oder basisch zu einer Verbindung der allgemeinen Formel I, in der Y für die Gruppe =NH steht, hydrolysiert und gegebenenfalls decarboxyliert.

Die saure Hydrolyse wird beispielsweise in einer wäßrigen Mineralsäure, wie Chlor- oder Bromwasserstoffsäure oder oder Schwefelsäure, und bei Temperaturen von 20 bis 100 °C durchgeführt. Gegebenenfalls kann es aber günstiger sein, z.B. wenn Y′ für die Gruppe
$=N-\underset{\underset{O}{\|}}{C}-OR^2$
steht, nichtwäßrige Reaktionsbedingungen anzuwenden. Solche schonenderen Methoden, die vorzugsweise bei Raumtemperatur durchgeführt werden, sind z.B. die Solvolyse mit Trifluoressigsäure in einem Chlorkohlenwasserstoff, wie Dichlormethan oder Chloroform, oder die Umsetzung mit Bromwasserstoff in Eisessig.

Die basische Hydrolyse erfolgt in einer verdünnten Lösung von Alkali- oder Erdalkalicarbonaten bzw. Alkali- oder Erdalkalihydroxiden in Wasser, niedrigen Alkoholen oder Gemischen von beiden und bei Temperaturen von Raumtemperatur bis zur Rückflußtemperatur des verwendeten Lösungsmittels.

Erfindungsgemäße Verbindungen der allgemeinen Formel I, bei denen A, B, R, R′ und X die oben angegebenen Bedeutungen besitzen und Y für ein Sauerstoffatom steht, können dadurch hergestellt werden, daß man eine Verbindung der allgemeinen Formel VI

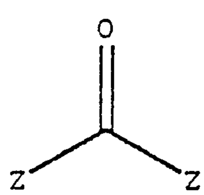

(VI)

in der Z für ein Halogenatom, eine Trichlormethoxygruppe oder den über ein Stickstoffatom gebundenen Rest eines Azols oder Benzazols mit mindestens zwei Stickstoffatomen im Fünfring steht, nacheinander mit einer Verbindung der allgemeinen Formel V

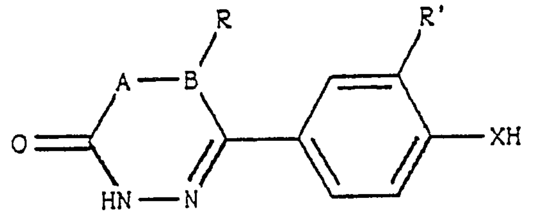

(V)

in der A, B, R, R′ und X wie oben definiert sind, und einer Verbindung der allgemeinen Formel III

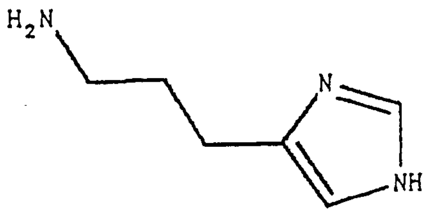

(III)

umsetzt.

Dabei ist die Reihenfolge der Umsetzungen der Verbindungen der allgemeinen Formel VI mit den Verbindungen der Formeln V und III beliebig, bevorzugt wird jedoch die Verbindung der Formel VI zunächst mit einer Verbindung der Formel V und dann mit der Verbindung der Formel III umgesetzt. Die Umsetzungen werden üblicherweise als Eintopf-Reaktion durchgeführt, d.h. die Zwischenstufen werden weder isoliert noch gereinigt.

Beispiele für die mit Z definierten Azole bzw. Benzazole sind der Imidazol-, 1,2,4-Triazol-, Tetrazol-, Benzimidazol-oder Benzotriazolring. Eine bevorzugte Verbindung der allgemeinen Formel VI ist N,N′-Carbonyldiimidazol. Die Umsetzungen werden in einem inerten Lösungsmittel, z.B. einem halogenierten Kohlenwasserstoff,wie Dichlormethan, einem Ether, z.B. Tetrahydrofuran, oder polaren Lösungsmitteln wie Acetonitril oder Dimethylformamid,durchgeführt. Die Reaktionstemperaturen können zwischen -20° C und dem Siedepunkt des verwendeten Lösungsmittels liegen. Steht in der allgemeinen Formel VI Z für ein Halogenatom, ist der Einsatz eines Säurefängers, wie z.B. eines tertiären Amins, wie Trimethylamin oder Pyridin, zweckmäßig.

Die nach den einzelnen Verfahrensvarianten erhaltenen Verbindungen werden in üblicher Weise isoliert und gereinigt, beispielsweise durch Umkristallisation, chromatographische Arbeitsweisen usw. Die bei den einzelnen Verfahrensvarianten erhaltenen Verbindungen können gegebenenfalls in ihr physiologisch annehmbares Salz umgewandelt werden. Diese Salze können z.B. mit Mineralsäuren, wie Chlor-, Brom-Iodwasserstoffsäure, Phosphorsäure, Metaphosphorsäure, Salpetersäure oder Schwefelsäure, oder mit organischen Säuren, wie Ameisensäure, Essigsäure, Propionsäure, Phenylessigsäure, Weinsäure, Zitronensäure, Fumarsäure, Methansulfonsäure, Embonsäure etc., gebildet werden.

Die erfindungsgemäßen Verbindungen der allgemeinen Formel I können sowohl in einer Reihe von tautomeren Formen als auch in mehreren stereoisomeren Formen vorliegen. Die Erfindung umfaßt daher neben den Salzen und Hydraten der oben beschriebenen Verbindungen der allgemeinen Formel I auch alle tautomeren und stereoisomeren Formen.

Die erfindungsgemäßen Verbindungen können zur Verabreichung in jeder beliebigen Weise formuliert werden.

Die Erfindung umfaßt daher auch Arzneimittel, die mindestens eine erfindungsgemäße Verbindung zur Verwendung in der Human-oder Veterinärmedizin enthalten. Solche Arzneimittel können herkömmlicherweise unter Verwendung eines oder mehrerer pharmazeutisch annehmbarer Träger oder Verdünnungsmittel hergestellt werden.

Die erfindungsgemäßen Verbindungen können daher für die orale, bukkale, topische, parenterale oder rektale Verabreichung formuliert werden.

Für die orale Verabreichung kann das Arzneimittel in Form von beispielsweise Tabletten, Kapseln, Pulvern, Lösungen, Sirups oder Suspensionen vorliegen, die unter Verwendung von annehmbaren Verdünnungsmitteln auf herkömmliche Weise hergestellt werden. Für die bukkale Verabreichung kann das Arzneimittel die Form von Tabletten oder Briefchen einnehmen, die in herkömmlicher Weise formuliert

werden.

Die erfindungsgemäßen Verbindungen können für die parenterale Verabreichung durch Bolusinjektion oder kontinuierliche Infusion formuliert werden. Formulierungen zur Injektion können in Dosiseinheitsform als Ampullen oder in Mehrfachdosenbehältern mit zugesetztem Konservierungsmittel vorliegen. Die Arzneimittel können solche Formen wie Suspensionen, Lösungen oder Emulsionen in öligen oder wäßrigen Trägern, einnehmen, und sie können Formulierungshilfsmittel, wie Suspendierungs-, Stabilisierungs- und/oder Dispergierungsmittel, enthalten.

Alternativ kann der Wirkstoff auch in Pulverform zur Rekonstitution mit einem geeigneten Träger, z.B. sterilem, pyrogenfreien Wasser, vor dem Gebrauch vorliegen.

Die erfindungsgemäßen Verbindungen können auch für rektale Zubereitungen, z.B. Suppositorien oder Retentionseinläufe, formuliert werden, die z.B. herkömmliche Suppositoriengrundlagen, wie Kakaobutter oder andere Glyceride, enthalten.

Zur topischen Anwendung können die erfindungsgemäßen Verbindungen als Salben, Cremes, Gels, Lotionen, Pulver oder Sprays in herkömmlicher Weise formuliert werden.

Für die orale Verabreichung ist eine geeignete Tagesdosis an erfindungsgemäßen Verbindungen 1 bis 4 Dosen bis insgesamt 5 mg bis 1 g/Tag, je nach Zustand des Patienten. Im Einzelfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es z.B. Fälle, in denen mit weniger als der oben genannten Mindestmenge ausgekommen werden kann, während in anderen Fällen die genannte obere Grenze überschritten werden muß.

Die erfindungsgemäßen Diazinderivate der allgemeinen Formel I zeigen ausgeprägte cardiovaskuläre, insbesondere cardiotone Wirkungen und eignen sich daher zur Behandlung und Vorbeugung von Erkrankungen des Herzens und des Kreislaufs.

So zeigen sie eine ausgezeichnete positiv inotrope Wirkung am narkotisierten Meerschweinchen nach intravenöser Applikation.

Hämodynamische Charakterisierung am narkotisierten Meerschweinchen (i. v.- Applikation)

a) Methode

Die Tiere werden mit Urethan (1.5 g/kg) narkotisiert. Zur volumenkontrollierten Beatmung wird die Trachea kanüliert. Danach erfolgt die operative Freilegung beider Karotiden; über die rechte Karotis wird ein Tip-Katheder (3F) eingeführt, der unter fortlaufender Druckregistrierung dann über die Aorta ascendens in den linken Ventrikel vorgeführt wird. Die erfolgreiche Passage der Aortenklappen wird hierbei durch die typische linksventrikuläre Druckkurve erkannt. Über die linke Karotis wird zur Thermodilution ein Thermistorfühler (3F, F. Edwards) in den Aortenbogen vorgeschoben. Der Thermistorfühler hat gleichzeitg ein Lumen zur arteriellen Blutdruckregistrierung. Zur Applikation des Kälteinjektats (0,2 ml 0,9 % NaCl, 15 °C) wird durch die rechte Vena jugularis ein Katheder vor den rechten Vorhof plaziert.

Alle Substanzen sind in physiologischer Kochsalzlösung gelöst und werden über die linke Vena jugularis infundiert (Infusionsvolumen 0,02 ml/min ; die Applikation erfolgt nach hämodynamischer Stabilisierung und unter $\beta$-Blockade (Metoprolol 2mg/kg i. m. . Alle Kreislaufparameter werden kontinuierlich auf einem Direktschreiber registriert. Die Kontraktilität (dp/dt) wird über die Volumenkurve berechnet.

b) Meßwerte

| Beispiel Nr. | Dosis µg/kg/min | Maximale Zunahme der Kontraktilität LV dp/dt |
|---|---|---|
| 1 | 5,0 | + 193 % |
| 2 | 10,0 | + 126 % |
| 3 | 10,0 | + 96 % |

Die folgenden Beispiele erläutern die Erfindung.

Alle Zwischenprodukte wurden routinemäßig auf die Reinheit durch Dünnschichtchromatographie überprüft, wobei für den Nachweis UV-Licht sowie Sprühreagenzien, wie Echtblausalz B/ Natronlauge, verwendet wurden.

Die Dünnschichtchromatographie wurde an Kieselgelfolien Polygram SIL G/$UV_{254}$ (Machery-Nagel) durchgeführt. Die präparative Chromatographie wurde unter Verwendung von Kieselgel Merck Art.Nr. 7734 und 7749 durchgeführt.

Für die Elutionsmittel wurden folgende Abkürzungen verwendet:

| | | |
|---|---|---|
| A | Dichlormethan: Methanol | 80:20 |
| B | Ethylacetat: Methanol:konz. Ammoniak | 80:18:2 |
| C | Dichlormethan:Methanol:konz. Ammoniak | 85:13:2 |
| D | Dichlormethan:Methanol:konz. Ammoniak | 60:30:10 |
| E | Ethylacetat:Puffer* | 60:40 |
| F | Ethylacetat:Puffer* | 50:50 |
| G | Dichlormethan:Methanol | 90:10 |
| H | Dichlormethan:Methanol:konz. Ammoniak | 90:9:1 |
| I | Dichlormethan:Acetonitril | 90:10 |

*Puffer: Methanol:konz. Ammoniak gesättigt mit Ammoniumchlorid 95:5

Herstellung der Ausgangsverbindung **5-(4-Chlor-3-nitro-phenyl)-6-methyl-3H,6H-1,3,4-thiadiazin-2-on**

29,3g (100 mmol) α-Brom-4-chlor-3-nitro-propiophenon und 14,2g (134 mmol) Hydrazinthiocarbonsäure-methylester werden in 400 ml abs. Ethanol mit 22g einer 23%-igen Lösung von Chlorwasserstoff in Isopropanol versetzt und 3,5 Stunden unter Rückfluß gekocht. Die erhaltene Lösung wird dann auf ca. 100 ml i. Vak. eingeengt und im Eisbad auf 2 °C abgekühlt. Der ausgefallene Feststoff wird abgesaugt und i. Vak. getrocknet. Durch weiteres Einengen der Mutterlauge und Verdünnen mit 50 ml Dichlormethan wird eine Zweitfraktion erhalten. Die Gesamtausbeute beträgt 25,6g (90 %) blaßgelbe Kristalle vom Schmp. 168 - 169 °C.

$C_{10}H_8ClN_3O_3S$ (285.71)

**Beispiel 1**

$N^1$-[3-(1H-Imidazol-4-yl)propyl]-$N^3$-[2-[[4-(6-methyl-2-oxo-3,6-dihydro-1,3,4-thiadiazin-5-yl)-2-nitro-phenyl]-amino]ethyl]-guanidin

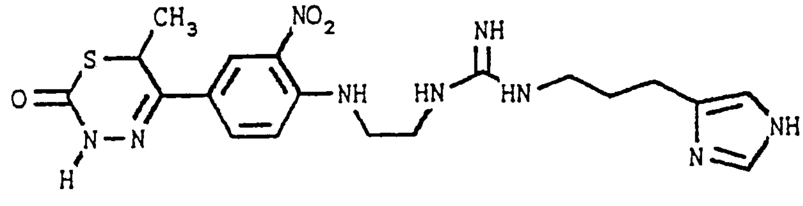

a) 5-[4-(2-Aminoethyl)amino-3-nitro-phenyl]-6-methyl-3H,6H-thiadiazin-2-on

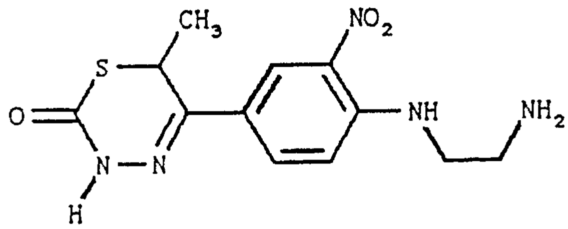

5,7 g (20 mmol) 5-(4-Chlor-3-nitro-phenyl)-6-methyl-3H,6H-1,3,4-thiadiazin-2-on und 4.0 ml (60 mmol) Ethylendiamin werden in 40 ml Dioxan 2 Stunden unter Rückfluß gekocht.

Das abgekühlte Reaktionsgemisch wird zu einer Lösung von 2 g Kaliumcarbonat in 50 ml Wasser gegeben und die wässrige Phase dreimal mit je 30 ml Chloroform/Methanol (80/20 v/v) extrahiert. Das nach Trocknen und Eindampfen der vereinigten organischen Phasen i. Vak. erhaltene Öl wird an Kieselgel mit Laufmittel A chromatographiert und ergibt nach Eindampfen der Hauptfraktion und Kristallisation des Rückstandes aus Methanol/Wasser 1,82 g (26 %) orangegelbe Kristalle vom Schmp. 165-168 °C.

$C_{12}H_{15}N_5O_3S$ (309,35)

Rf = 0,27 (Laufmittel B)

b) S-Methyl-N-[2-[[4-(6-methyl-2-oxo-3,6-dihydro-1,3,4-thiadiazin-5-yl)-2-nitro-phenyl]-amino]ethyl]-isothiuroniumjodid

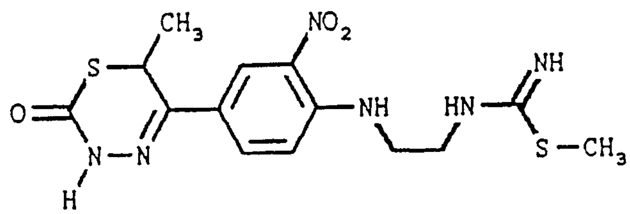

x IH

1,40 g (4,5 mmol) 5-[4-(2-Aminoethyl)amino-3-nitro-phenyl]-6-methyl-3H,6H-thiadiazin-2-on und 1,24 g (5 mmol) Dithiocarbimidsäure-S,S-dimethylester-hydrojodid werden in 60 ml Acetonitril 8 Stunden unter Rückfluß gekocht.

Das nach Abdampfen des Lösungsmittels i. Vak. erhaltene Rohprodukt wird an Kieselgel mit Laufmittel A chromatographiert und ergibt nach Eindampfen der Hauptfraktion i. Vak. 1,62 g (71 %) eines orangegelben, amorphen Feststoffes.

$C_{14}H_{19}JN_6O_3S_2$ (510,38)

Rf = 0,38 (Laufmittel C)

c) $N^1$-[3-(1H-Imidazol-4-yl)propyl]-$N^3$-[2-[[4-(6-methyl-2-oxo-3,6-dihydro-1,3,4-thiadiazin-5-yl)-2-nitro-phenyl]-amino]ethyl]-guanidin

1,60 g (3,13 mmol) des unter b) erhaltenen Isothiuroniumjodids und 0,395 g (3,13 mmol) 3-(1H-Imidazol-4-yl)propylamin werden in 30 ml Dimethylformamid 3 Tage bei Raumtemperatur gerührt. Das Reaktionsgemisch wird i. Vak. eingeengt, dann mit 20 ml einer 20%-igen Kaliumcarbonat-Lösung versetzt und mit 3 x 30 ml Chloroform-Methanol-Gemisch (80/20 v/v) extrahiert. Die vereinigten organischen Phasen werden getrocknet, filtriert und i.Vak. eingedampft.

Zweimalige chromatographische Reinigung des Rohprodukts an Kieselgel mit den Laufmitteln D und E ergibt 0,15 g (10 %) eines orangeroten, amorphen Pulvers, das bei 135-145 °C unscharf schmilzt.

$C_{19}H_{25}N_9O_3S$ (459,53)

Rf = 0,48 (Laufmittel F)

$^1$H-NMR-Daten (DMSO-$d_6$,TMS als interner Standard $\delta$ =

1.37 (d) 3H

1,68 (quin) 2H
2,45 (m) 2H
2,9-3,6 (m) 6H
4,64 (q) 1H
6,69 (s) 1H
7,19 (d) 1H
7,45 (s) 1H
7,93 (dd) 1H
8,36 (d) 1H
8,6 (breit) 5H,
austauschbar mit $D_2O$
ppm.

**Beispiel 2**

N[1]-[3-(1H-Imidazol-4-yl)propyl]-N[3]-[3-[[4-(6-methyl-2-oxo-3,6-dihydro-1,3,4-thiadiazin-5-yl)-2-nitro-phenyl]-amino]propyl]-guanidin

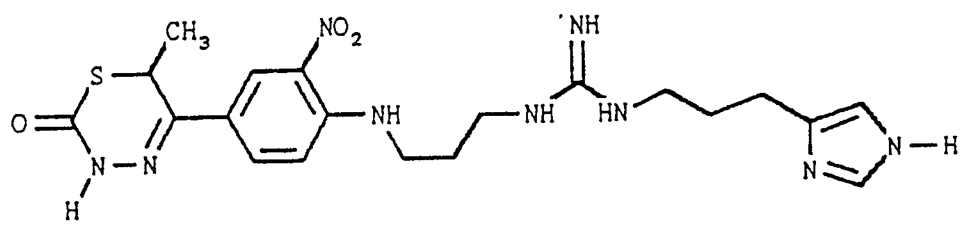

a) 5-[4-(3-Aminopropyl)amino-3-nitro-phenyl]-6-methyl-3H,6H-1,3,4-thiadiazin-2-on

Analog zu Beispiel 1a) werden aus 8,57 g (30 mmol) 5-(4-Chlor-3-nitro-phenyl)-6-methyl-3H,6H-1,3,4-thiadiazin-2-on und 7,5 ml (90 mmol) 1,3-Diaminopropan 7,27 g (75 %) eines orangeroten Feststoffes vom Schmp. 192-193 °C erhalten.
$C_{13}H_{17}N_5O_3S$ (323,38)
Rf = 0,43 (Laufmittel C)

b) S-Methyl-N-[3-[[4-(6-methyl-2-oxo-3,6-dihydro-1,3,4-thiadiazin-5-yl)-2-nitro-phenyl]amino]propyl]-isothiuroniumjodid

Entsprechend Beispiel 1b) werden aus 1,60 g (5 mmol) 5-[4-(3-Aminopropyl)amino-3-nitro-phenyl]-6-methyl-3H,6H-1,3,4-thiadiazin-2-on und 1,37 g (5,5 mmol) Dithiocarbimidsäure-S,S-dimethylester-hydrojodid 1,4 g (53 %) eines orangeroten, amorphen Feststoffes erhalten.
$C_{15}H_{21}JN_6O_3S_2$ (524,41)
Rf = 0,4 (Laufmittel A)

c) N[1]-[3-(1H-Imidazol-4-yl)propyl]-N[3]-[3-[[4-(6-methyl-2-oxo-3,6-dihydro-1,3,4-thiadiazin-5-yl)-2-nitro-phenyl]-amino]propyl]-guanidin

Die Titelverbindung erhält man aus 1,26 g (2,5 mmol) des unter b) erhaltenen Isothiuroniumsalzes und 0,32 g (2,6 mmol) 3-(1H-Imidazol-4-yl)-propylamin analog zu Beipiel 1c).
Das chromatographisch gereinigte Produkt kristallisiert aus Ethanol mit einer Ausbeute von 0,21 g (18 %) und einem Schmelzbereich von 139-143 °C als ziegelroter Feststoff.
$C_{20}H_{27}N_9O_3S$ (473,56)
Rf = 0,44 (Laufmittel F)

$^1$H-NMR-Daten (DMSO-$d_6$,TMS als interner Standard) $\delta$ =
1,37 (d) 3H
1,61-1,96 (m) 4H
2,48 (t) 2H
3,0-3,6 (m) 6H
4,55 (q) 1H
6,72 (s) 1H
7,12 (d) 1H
7,50 (s) 1H
7,92 (dd) 1H
8,38 (d) 1H
8,5 (breit) 5H,
austauschbar mit $D_2O$
ppm.

**Beispiel 3**

5-[4-[4-[3-(1H-Imidazol-4-yl)propylamino-iminomethylen]-piperazin-1-yl]-3-nitro-phenyl]-6-methyl-3H,6H-1,3,4-thiadiazin-2-on

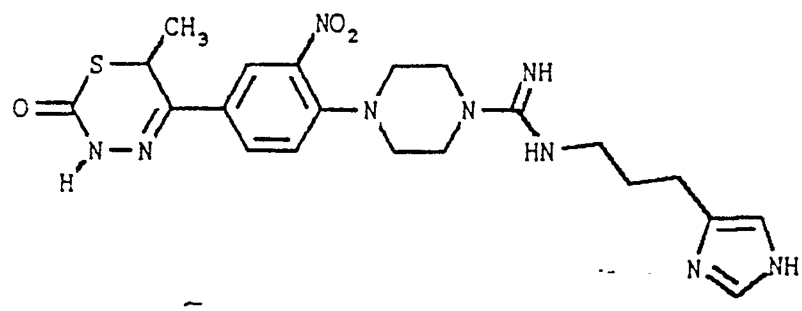

a) 5-[4-(Piperazin-1-yl)-3-nitro-phenyl]-6-methyl-3H,6H-1,3,4-thiadiazin-2-on

9,0 g (31,5 mmol) 5-(4-Chlor-3-nitro-phenyl)-6-methyl-3H,6H-1,3,4-thiadiazin-2-on und 8,1 g (94,4 mmol) Piperazin werden in 50 ml Dioxan 2 Stunden unter Rückfluß gekocht.
Das abgekühlte Reaktionsgemisch wird filtriert, das Filtrat i. Vak. eingedampft und der erhaltene Rückstand aus Methanol umkristallisiert.
Man erhält 8,6 g (81 %) orangerote Kristalle vom Schmp. 190-192 °C.
$C_{14}H_{17}N_5O_3S$ (335,39)
Rf = 0,47 (Laufmittel C)

b) 5-[4-[4-Methylthio-iminomethylen-piperazin-1-yl]-3-nitro-phenyl]-6-methyl-3H,6H-1,3,4-thiadiazin-2-on-hydrojodid

8,0 g (24 mmol) 5-[4-(Piperazin-1-yl)-3-nitro-phenyl]-6-methyl-3H,6H-1,3,4-thiadiazin-2-on und 9,7 g (39 mmol) Dithiocarbimidsäure-S,S-dimethylester-hydrojodid werden in 180 ml Acetonitril 13 Stunden unter Rückfluß gekocht.
Nach Abkühlen auf Raumtemperatur wird das unumgesetzte Ausgangsmaterial abgesaugt und das Filtrat i. Vak. eingedampft. Das Rohprodukt wird an Kieselgel mit Laufmittel G chromatographiert und ergibt nach Abdampfen der Lösungsmittel und Kristallisation der Produktfraktionen mit Ethylacetat Methanol 4,25 g (33 %) orangerote Kristalle vom Schmp. 193-196 °C.
$C_{16}H_{21}JN_6O_3S_2$ (536,42)

Rf = 0,62 (Laufmittel H)

c) 5-[4-[4-[3-(1H-Imidazol-4-yl)propylamino-iminomethylen]-piperazin-1-yl]-3-nitro-phenyl]-6-methyl-3H,6H-1,3,4-thiadiazin-2-on

2,52 g (4,7 mmol) des unter b) erhaltenen Isothiuroniumjodids und 0,64 g (5,1 mmol) 3-(1H-Imidazol-4-yl)-propylamin werden in 25 ml Dimethylformamid 30 Stunden bei Raumtemperatur gerührt.
Das Reaktionsgemisch wird dann i. Vak. weitgehend eingeengt, der Rückstand mit 20 ml gesättigter Kaliumcarbonatlösung versetzt und zweimal mit 50 ml Dichlormethan/ Methanol (80/20 v/v) extrahiert. Die vereinigten organischen Phasen werden mit Kaliumcarbonat getrocknet und i. Vak. eingedampft.
Das so erhaltene Rohprodukt wird an Kieselgel mit Laufmittel F chromatographiert. Die Produktfraktionen werden vereinigt, mit 10%-iger Kaliumcarbonatlösung zweimal extrahiert, getrocknet und i. Vak. eingedampft.
Nach Verrühren des Rückstandes mit Ethylacetat erhält man 0,68 g (30 %) eines amorphen, orangegelben Feststoffes.
$C_{21}H_{27}N_9O_3S$ (485,57)
Rf = 0,32 (Laufmittel E)
$^1$H-NMR-Daten (DMSO-$d_6$,TMS als interner Standard) $\delta$ =
1,46 (d) 3H
1,62 (quin) 2H
2,56 (t) 2H
3,0-3,8 (m) 10H
4,79 (q) 1H
6,79 (s) 1H
7,39 (d) 1H
7,55 (s) 1H
8,01 (d) 1H
8,25 (s) 1H
9,5 (breit) 3H,
austauschbar mit $D_2O$
ppm.

## Beispiel 4

N[1]-[3-(1H-Imidazol-4-yl)propyl]-N[2]-tert.-butoxycarbonyl-N[3]-[1-[4-(6-methyl-2-oxo-3,6-dihydrol,3,4-thiadiazin-5-yl)-2-nitro-phenyl] piperidin-4-yl]-guanidin

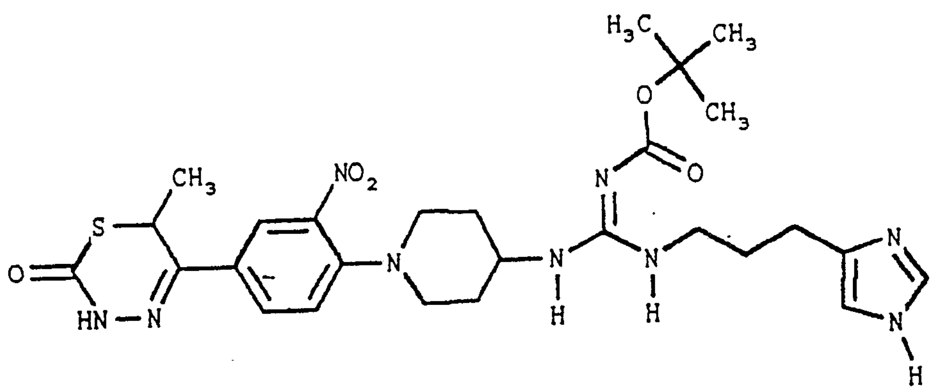

a) 5-[4-(4-Phthalimido-piperidin-1-yl)-3-nitro-phenyl]-6-methyl-3H,6H-1,3,4-thiadiazin-2-on

7,79 g (27,3 nmol) 5-(4-Chlor-3-nitro-phenyl)-6-methyl-3H,6H-1,3,4-thiadiazin-2-on, 6,91 g (30,0 mmol) 4-Phthalimido-piperidin und 4.2 ml (30 mmol) Triethylamin werden in 150 ml Dioxan 8 Stunden unter Rückfluß gekocht.
Nach Abkühlen wird die erhaltene Suspension i. Vak. eingedampft, der Rückstand in 100 ml Wasser aufgenommen und 10 min gerührt. Der entstandene Feststoff wird abgesaugt und i. Vak. bei 75 °C getrocknet.
Man erhält 12,2 g (93 %) orangegelbe Kristalle vom Schmp. 267-269 °C.
$C_{23}H_{21}N_5O_5S$
Rf = 0,50 (Laufmittel I)

b) 5-[4-(4-Aminopiperidin-1-yl)-3-nitro-phenyl]-6-methyl-3H,6H-1,3,4-thiadiazin-2-on

8,0 g (16,7 mmol) der unter a) erhaltenen Phthalimidverbindung werden in 110 ml Ethanol mit 4,0 ml (83,5 mmol) Hydrazinhydrat eine Stunde unter Rückfluß gekocht.
Nach Abkühlen auf Raumtemperatur wird der ausgefallene Feststoff abgesaugt und das Filtrat i. Vak. eingedampft. Der erhaltene Rückstand wird in 100 ml Wasser verrührt und der dabei ausfallende Feststoff abgesaugt und i. Vak. getrocknet.
Es werden 4.65 g (80 %) eines orangeroten Feststoffes vom Schmp. 154-157 °C erhalten.
$C_{15}H_{19}N_5O_3S$ (349,41)
Rf = 0,39 (Laufmittel F)

c) N'-[3-(1H-Imidazol-4-yl)propyl]-N²-tert.-butoxycarbonyl-N³-[1-[4-(6-methyl-2-oxo-3,6-dihydro-1,3,4-thiadiazin-5-yl)-2-nitrophenyl]piperidin-4-yl]-guanidin

1,00 g (2,86 mmol) des unter b) erhaltenen Amins werden mit 0,90 g (2,87 mmol) N-(Tert.-Butoxycarbonyl)-imidokohlensäurediphenylester in 20 ml Acetonitril 2 Stunden bei Raumtemperatur gerührt.
0,36 g (2,86 mmol) 3-(1H-Imidazol-4-yl)-propylamin werden zugegeben und das Reaktionsgemisch 12 Stunden unter Rückfluß gekocht. Die abgekühlte Lösung wird i. Vak. eingedampft und der Rückstand an Kieselgel mit Laufmittel H chromatographiert. Die Produktfraktionen ergeben nach Einengen i. Vak. einen orangefarbenen Schaum, der nach Aufkochen mit 20 ml Acetonitril kristallisiert.
Man erhält 0.84 g (49 %) orangegelbe Kristalle vom Schmp. 221-223 °C.
$C_{27}H_{37}N_9O_5S$ (599,72)
Rf = 0,5 (Laufmittel H)
$^1$H-NMR-Daten (DMSO-$d_6$,TMS als interner Standard) $\delta$ =
1,38 (s) 9H
1,46 (d) 3H
1,5-2,0 (m) 6H
2,50 (t) 2H
2,9-3,4 (m) 7H
4,79 (q) 1H
6,78 (s) 1H
7,37 (d) 1H
7,52 (s) 1H
7,96 (dd) 1H
8,20 (d) 1H
9,00 (breit) 2H,
austauschbar mit $D_2O$
ppm.

**Beispiel 5**

5-[4-[4-[3-(1H-Imidazol-4-yl)propylamino-(tert.-butoxycarbonyl)iminomethylen]-piperazin-1-yl]-3-nitro-phenyl]-6-methyl-3H,6H-1,3,4-thiadiazin-2-on

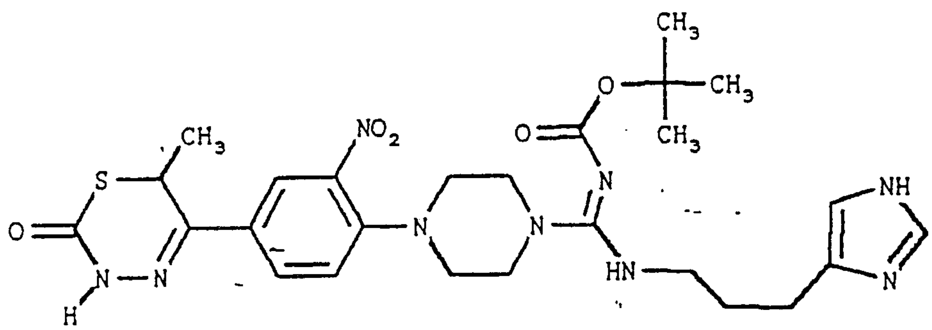

9,0 g (26,8 mmol) 6-Methyl-5-[4-(piperazin-1-yl)-3-nitro-phenyl]-3H,6H-1,3,4-thiadiazin-2-on und 8,4 g (26,8 mmol) N-(Tert.-Butoxycarbonyl)-imidokohlensäurediphenylester werden in 60 ml Acetonitril 16 Stunden bei Raumtemperatur gerührt. Nach Zugabe von 3,4 g (26,8 mmol) 3-(1H-Imidazol-4-yl)-propylamin wird das Reaktionsgemisch dann 8 Stunden unter Rückfluß gekocht. Das nach Abdampfen des Lösungsmittels i. Vak. erhaltene Öl wird an Kieselgel mit Laufmittel G chromatographiert.
Aus der Hauptfraktion erhält man nach Eindampfen i. Vak. 10,2 g (65 %) eines orangegelben, amorphen Feststoffes.

$C_{26}H_{35}N_9O_5S$ (585,69)

Rf = 0,3 (Laufmittel H)

$^1$H-NMR-Daten (DMSO-$d_6$ TMS als interner Standard) $\delta$ =
1,37 (s) 9H
1,47 (d) 3H
1,78 (m) 2H
2,45-2,6 (m) 2H
3,0-3,55 (m) 10H
4,80 (q) 1H
6,74 (s) 1H
7,37 (d) 1H
7,53 (s) 1H
7,99 (dd) 1H
8,24 (d) 1H
11,76 (s) 1H,
austauschbar mit $D_2O$
ppm.

## Beispiel 6

3-Cyano-5-[4-[4-[3-(1H-imidazol-4-yl)propylamino-(tert.-butoxy carbonyl)iminomethylen]-piperazin-1-yl]-phenyl]-6-methyl-3H,6H-1,3,4-thiadiazin-2-on

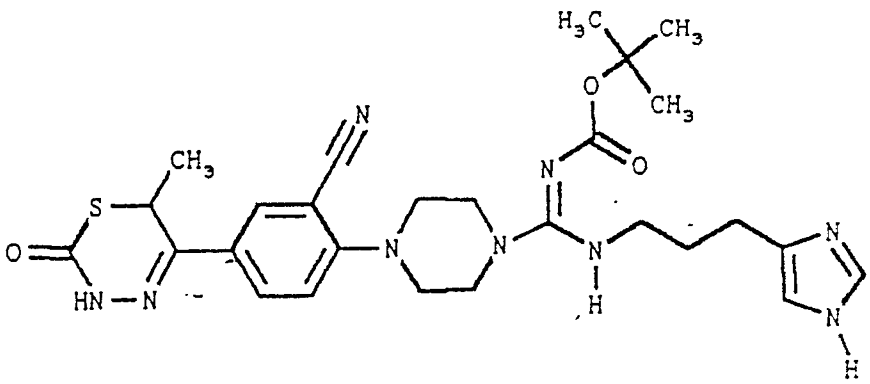

1,5 g (4,8 mmol) 5-[3-Cyano-4-(piperazin-1-yl)-phenyl]-6-methyl-3H, 6H-1,3,4-thiadiazin-2-on und 1,5 g (4,8 mmol) N-(Tert.-butoxycarbonyl)-imidokohlensäurediphenylester werden in 25 ml Acetonitril 6 Stunden bei 40 °C gerührt.

0,6 g (4,8 mmol) 3-(1H-Imidazol-4-yl)-propylamin werden zugesetzt und das Reaktionsgemisch 9 Stunden unter Rückfluß gekocht.

Das nach Abdampfen des Lösungsmittels i. Vak. erhaltene Rohprodukt wird an Kieselgel zunächst mit Laufmittel G und ein zweites Mal mit Laufmittel H chromatographisch gereinigt.

Man erhält 0,35 g (13 %) der Titelverbindung in Form eines beigen, amorphen Feststoffes.

$C_{27}H_{35}N_9O_3S$ (565,70)

Rf = 0,5 (Laufmittel A)

$^1$H-NMR-Daten (DMSO-$d_6$,TMS als interner Standard) $\delta$ =

1,37 (s) 9H
1,46 (d) 3H
1,78 (m) 2H
2,51 (t) 2H
3,0-3,6 (m) 10H
4,77 (q) 1H
6,73 (s) 1H
7,24 (d) 1H
7,51 (s+breit) 2H
1H austauschbar mit $D_2O$
8,03 (dd) 1H
8,10 (d) 1H
11,78 (s) 1H
austauschbar mit $D_2O$
ppm.

**Beispiel 7**

N′-[3-(1H-Imidazol-4-yl)propyl]-N³-[1-[4-(6-methyl-2-oxo-3,6-dihydro-1,3,4-thiadiazin-5-yl)-2-nitro-phenyl]-piperidin-4-yl]-guanidin

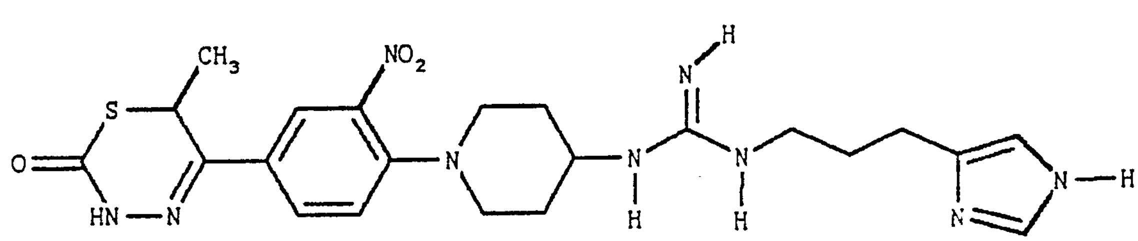

1,7g (2,8 mmol) $N^1$-[3-(1H-Imidazol-4-yl)propyl]-$N^2$-tert.-butoxycarbonyl-$N^3$-[1-[4-(6-methyl-2-oxo-3,6-dihydro-1,3,4-thiadiazin-5-yl)-2-nitro-phenyl]-piperidin-4-yl]-guanidin (Beispiel 4) werden in 50 ml Dichlormethan gelöst, mit 2,1 ml (28 mmol) Trifluoressigsäure versetzt und 2 Tage bei Raumtemperatur gerührt. Danach wird das Lösungsmittel i. Vak. bei 20 °C abgedampft, der Rückstand in 10 ml gesättigter Kaliumcarbonat-Lösung aufgenommen und mit 3x10 ml Isopropanol extrahiert. Die vereinigten organischen Phasen werden eingeengt und der Rückstand zweimal an Kieselgel chromatographiert, zunächst mit Laufmittel F und beim zweiten Mal mit Laufmittel E.

Die Produktfraktionen werden jeweils eingeengt, in gesättigter Kaliumcarbonat-Lösung aufgenommen und dreimal mit Isopropanol extrahiert.

Der nach Trocknen und Eindampfen der vereinigten organischen Phasen erhaltene Feststoff wird in 20 ml Wasser verrührt, abgesaugt und aus 45 ml Ethanol umkristallisiert. Man erhält 0,72g (51 %) eines orangeroten Feststoffes vom Schmp. 193-195 °C.

$C_{22}H_{29}N_9O_3S$ (499,60)

Rf = 0,36 (Laufmittel E)

$^1$H-NMR-Daten (DMSO-$d_6$, TMS als interner Standard) δ =

1,33 (d) 3H
1,5-2,0 (m) 6H
2,55 (t) 2H
2,8-3,7 (m) 7H
4,44 (q) 1H
6,77 (s) 1H
7,27 (d) 1H
7,53 (s) 1H
7,96 (dd) 1H
8,0 (breit) 4H,
austauschbar mit $D_2O$
8,10 (d) 1H
ppm.

## Beispiel 8

5-[4-[4-[3-(1H-Imidazol-4-yl)propylamino-(tert.-butoxy-carbonyl)iminomethylen]-piperazin-1-yl]-3-nitrophenyl]-3H,6H-1,3,4-thiadiazin-2-on

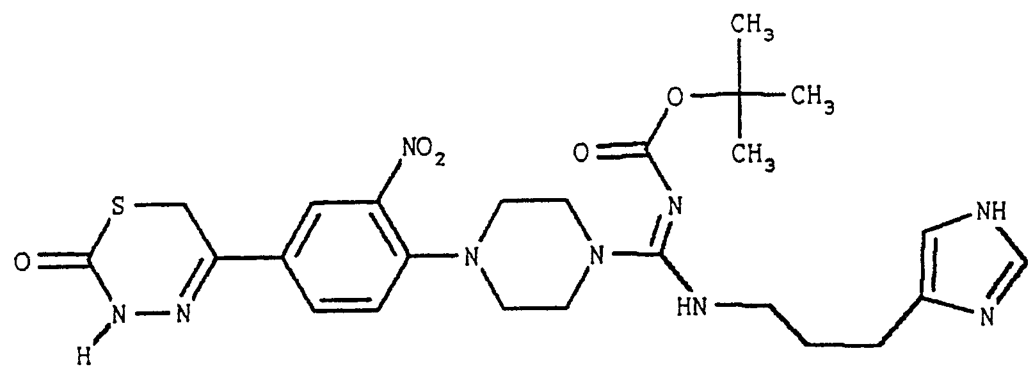

Ausgehend von 4,0g (12,5 mmol 5-[4-(Piperazin-1-yl)-3-nitrophenyl]-3H,6H-1,3,4-thiadiazin-2-on, 3,9g (12,5 mmol) N-(Tert.-Butoxycarbonyl)-imidokohlensäurediphenylester und 1,56g (12,5 mmol) 3-(1H-Imidazol-4-yl)propylamin werden analog zu Beispiel 5 nach chromatographischer Reinigung mit Laufmittel C 1,5g (21%) eines orangegelben, amorphen Feststoffes erhalten.

$C_{25}H_{33}N_9O_5S$ (571,66)

$^1$H-NMR-Daten (DMSO-$d_6$, TMS als interner Standard) δ =

1,36 (s) 9H
1,77 (quin) 2H

2,50 (t) 2H
2,9-3,7 (m) 10H
4,24 (s) 2H
6,73 (s) 1H
7,37 (d) 1H
7,51 (s) 1H
7,98 (dd) 1H
8,24 (d) 1H
11,65 (s) 1H,
austauschbar mit $D_2O$
11,8 (breit) 2H,
austauschbar mit $D_2O$
ppm.

**Beispiel 9**

5-[4-[4-[3-(1H-Imidazol-4-yl)propylamino-iminomethylen]-piperazin-1-yl]-3-nitro-phenyl]-3H,6H-1,3,4-thiadiazin-2-on

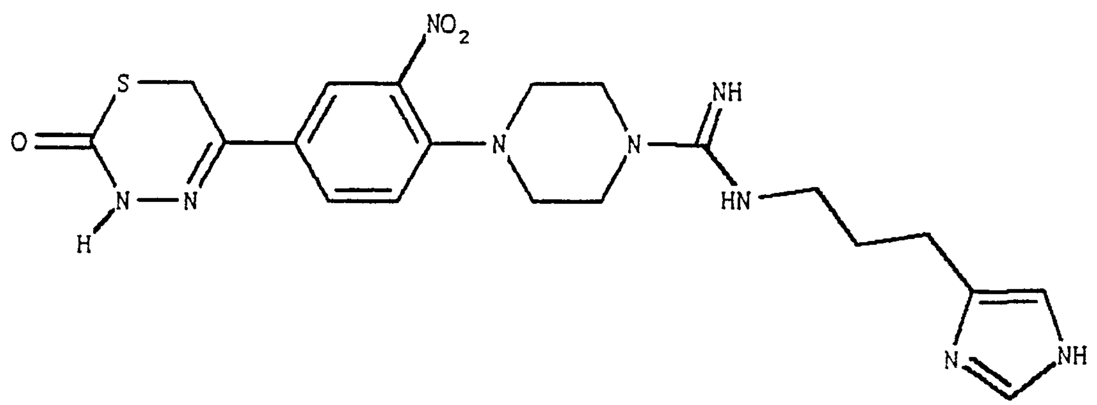

Analog zu Beispiel 7 erhält man aus 3,0g (5,2 mmol) 5-[4-[4-[3-(1H-Imidazol-4-yl)propylamino-(tert.-butoxycarbonyl)iminomethylen]-piperazin-1-yl]-3-nitro-phenyl]-3H,6H-1,3,4-thiadiazin-2-on (Beisp. 8) 0,78g (32%) eines orangen, amorphen Feststoffes.
$C_{20}H_{25}N_9O_3S$ (471,54)
Rf = 0,4 (Laufmittel B)
$^1$H-NMR-Daten ($CD_3OD$, TMS als interner Standard) $\delta$ =
1,89 (quin) 2H
2,64 (t) 2H
3,0-3,7 (m) 10H
4,13 (s) 2H
4,9 (breit) 4H,
austauschbar mit $D_2O$
6,82 (s) 1H
7,31 (d) 1H
7,62 (s) 1H
7,99 (dd) 1H
8,24 (d) 1H
ppm.

**Beispiel 10**

$N^1$-[3-(1H-Imidazol-4-yl)propyl]-$N^2$-tert.-butoxycarbonyl-$N^3$-[1-[4-(2-oxo-3,6-diyhdro-1,3,4-thiadiazin-5-yl)-2-nitro-phenyl]-piperidin-4-yl]-guanidin

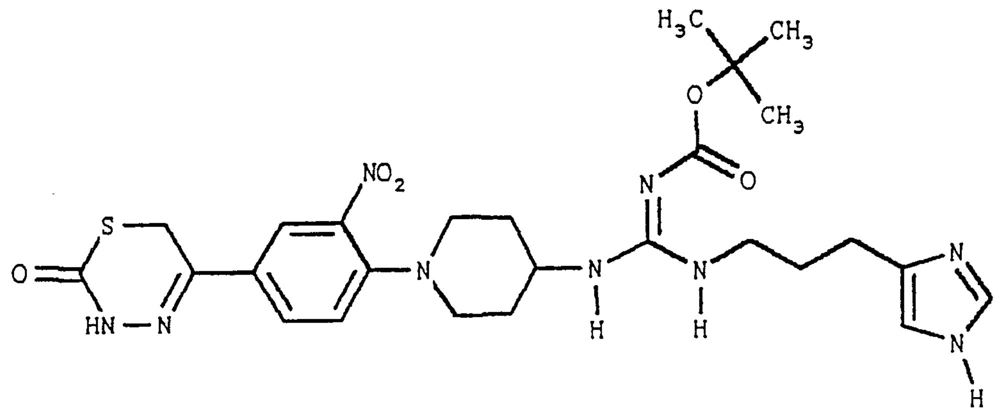

Entsprechend zu Beispiel 4c) werden aus 1,0g (2,98 mmol) 5-[4-(4-Aminopiperidin-1-yl)-3-nitro-phenyl]-3H,6H-1,3,4-thiadiazin-2-on, 0,93g (2,98 mmol) N-(Tert.-butoxycarbonyl -imidokohlensäurediphenylester und 0,37g (2,98 mmol) 3-(1H-Imidazol-4-yl)propylamin in 20 ml Dimethylformamid nach Chromatographie an Kieselgel mit Laufmittel H 0,65g (37%) eines orangegelben Feststoffes erhalten.

$C_{26}H_{35}N_9O_5S$ (585,69)

Rf = 0,45 (Laufmittel H)

$^1$H-NMR-Daten (DMSO-$d_6$,TMS als interner Standard) $\delta$ =

1,37 (s) 9H
1,5-2,0 (m) 6H
2,50 (t) 2H
2,9-3,4 (m) 7H
4,22 (s) 2H
6,77 (s) 1H
7,37 (d) 1H
7,51 (s) 1H
7,96 (dd) 1H
8,23 (d) 1H
11,75 (s) 1H,
austauschbar mit $D_2O$
11,8 (breit) 2H,
austauschbar mit $D_2O$
ppm.

*Beispiel 11*

**5-[4-[1-[3-(1H-Imidazol-4-yl)propylamino-(tert.-butoxycarbonyl)iminomethylen]-piperidin-4-yl]amino-3-nitrophenyl]-6-methyl-3H,6H-1,3,4-thiadiazin-2-on**

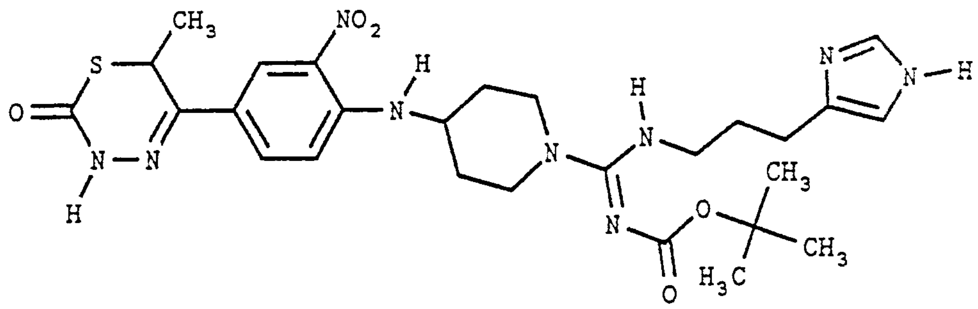

**a) 5-[4-[1-[Phenoxy-(tert.-butoxycarbonyl)iminomethylen] piperidin-4-yl]amino-3-nitrophenyl]-6-methyl-3H,6H-1,3,4-thiadiazin-2-on**

0,80g (2,28 mmol) 5-[4-(Piperidin-4-yl)amino-3-nitrophenyl]-6-methyl-3H,6H-1,3,4-thiadiazin-2-on und 0,80g (2,53 mmol) N-tert.-Butoxycarbonyl-diphenylimidocarbonat werden in 30 ml Acetonitril 2 Stunden unter Rückfluß gekocht. Nach Abdampfen des Lösungsmittels i.Vak. wird das erhaltene Rohprodukt an Kieselgel mit Laufmittel H chromatographiert. Die Hauptfraktion ergibt nach Eindampfen i.Vak. einen orangegelben Schaum, der beim Verrühren mit Methanol 0,80g (62%) orange Kristalle vom Schmp. 207-208 °C ergibt.
$C_{27}H_{32}N_6O_5S$ (568,66)
Rf = 0,68 (Laufmittel H)

**b) 5-[4-[1-[3-(1H-Imidazol-4-yl)propylamino-(tert.-butoxycarbonyl)iminomethylen]-piperidin-4-yl]-amino-3-nitrophenyl]-6-methyl-3H,6H-1,3,4-thiadiazin-2-on**

0,75g (1,31 mmol) der unter a) erhaltenen Verbindung werden mit 0,17g (1,35 mmol) 3-(1H-Imidazol-4-yl)propylamin in 30 ml Acetonitril 10 h zum Rückfluß erhitzt. Nach Abkühlen wird die Lösung i.Vak. eingeengt und der Rückstand an Kieselgel mit Laufmittel H gereinigt. Die Hauptfraktion mit dem Rf-Wert 0,37 ergibt nach Einkampfen i.Vak. 0,43g (55%) eines orangegelben, amorphen Feststoffes.
$C_{27}H_{37}N_9O_5S$ (599,72)
Rf = 0,37 (Laufmittel H)
$^1$H-NMR-Daten (DMSO-$d_6$, TMS als interner Standard) $\delta$ =
1,37 (s) 9H
1,46 (d) 3H
1,5-2,1 (m) 6H
2,51 (t) 2H
2,9-3,2 (m) 4H
3,7-4,0 (m) 3H
4,81 (q) 1H
6,73 (s) 1H
7,33 (d) 1H
7,50 (s) 1H
7,99 (dd) 1H
8,17 (d) 1H,
austauschbar mit $D_2O$
8,47 (d) 1H
11,67 (s) 1H,
austauschbar mit $D_2O$
ppm.

*Beispiel 12*

**5-[4-[1-[3-(1H-Imidazol-4-yl)propylamino-iminomethylen]-piperidin-4-yl]amino-3-nitrophenyl]-3H,6H-1,3,4-thiadiazin-2-on**

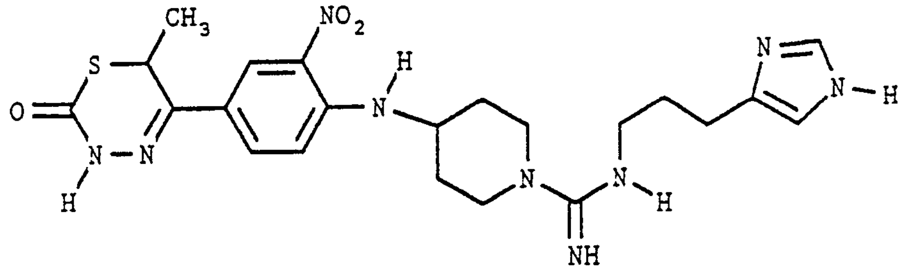

Analog zu Beispiel 7) erhält man aus 0,43g (0,71 mmol) 5-[4-[1-[3-(1H-Imidazol-4-yl)propylamino-(tert.-butoxycarbonyl)iminomethylen]-piperidin-4-yl]amino-3-nitrophenyl]-6-methyl-3H,6H-1,3,4-thiadiazin-2-on mit Trifluoressigsäure in Dichlormethan und nach chromatographischer Reinigung mit Laufmittel C 0,28g (79%) eines gelborangen, amorphen Feststoffes.

$C_{22}H_{29}N_9O_3S$ (499,60)

Rf = 0,42 (Laufmittel F)

$^1$H-NMR-Daten (DMSO-$d_6$-TMS als interner Standard) $\delta$ =

1,43 (d) 3H
1,4-2,05 (m) 6H
2,54 (t) 2H
2,8-3,1 (m) 4H
3,7-4,0 (m) 3H
4,70 (q) 1H
6,71 (s) 1H
7,29 (d) 1H
7,50 (s) 1H
7,7 (breit) 4H,
austauschbar mit $D_2O$
8,01 (dd) 1H
8,17 (d) 1H,
austauschbar mit $D_2O$
8,43 (d) 1H
ppm.

## Beispiel 13

**3-[4-[4-[3-(1H-Imidazol-4-yl)propylamino-iminomethylen]-piperazin-1-yl]-3-nitrophenyl]-4,5-dihydro-1,2,4-triazin-6(1H)-on**

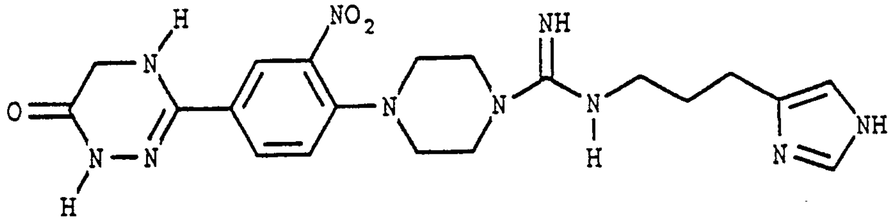

Zu einer Suspension von 0,85g (1,68 mmol) 3-[4-[4-(Methylthioiminomethylen)-piperazin-1-yl]-3-nitrophenyl]-4,5-dihydro-1,2,4-triazin-6(1H)-on in 20 ml DMF werden 0,23g (1,83 mmol) 3-(1H-Imidazol-4-yl)-propylamin gegeben und das Reaktionsgemisch 36h bei 50° C gerührt. Der entstandene Niederschlag wird abgesaugt, getrocknet und mit Methanol/Dichlormethan/konz. Ammoniak im Verhältnis 10:5:2 als Laufmittel an Kieselgel chromatographiert. Die sauberen Produktfraktionen (Rf= 0,43) werden vereinigt und i.Vak. eingedampft. Der erhaltene Rückstand wird mit absolutem Ethanol kristallisiert und ergibt 0.26g (25%) eines orangegelben, amorphen Feststoffes.

$C_{20}H_{26}N_{10}O_3$ (454,50)

Rf= 0,43 (Laufmittel:Methanol/Dichlormethan/konz. Ammoniak 10:5:2)

$^1$H-NMR-Daten (DMSO-$d_6$,TMS als interner Standard) $\delta$ =

1,84 (quin) 2H
2,56 (t) 2H
3,0-3,8 (m) 12H
6,78 (s) 1H
7,23 (d) 1H
7,55 (s) 1H
8,02 (dd) 1H
8,1 (breit) 2H, austauschbar mit $D_2O$
8,35 (d) 1H
8,6 (breit) 3H, austauschbar mit $D_2O$
ppm.

## Ansprüche

1. Diazinderivate der allgemeinen Formel I

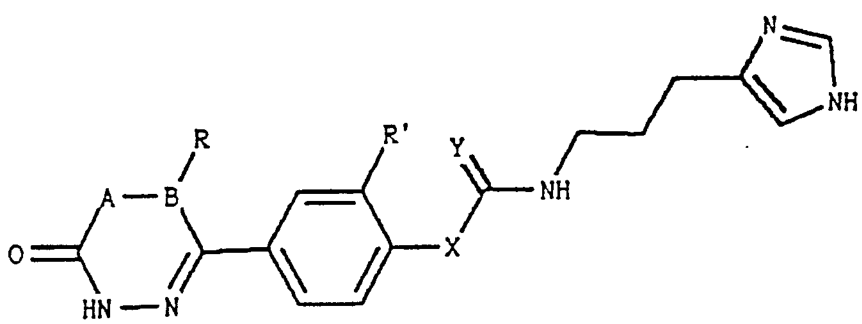

(I)

in der A für ein Schwefelatom und B für ein Kohlenstoffatom steht, oder eines der beiden Atome A und B ein unsubstituiertes oder mit einer $C_1$-$C_3$-Alkylgruppe substituiertes Stickstoffatom und das andere ein Kohlenstoffatom bedeuten, R ein Wasserstoffatom, eine $C_1$-$C_3$-Alkylgruppe oder eine Hydroxymethylgruppe und R′ ein Wasserstoffatom, eine Nitrogruppe, eine Cyanogruppe oder ein Halogenatom bedeutet, X für eine der Gruppen

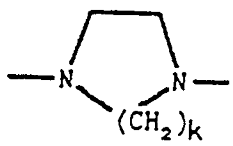

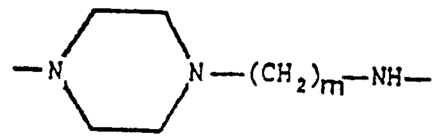

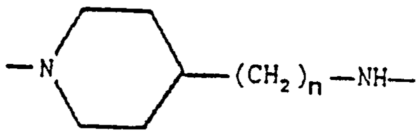

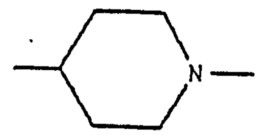

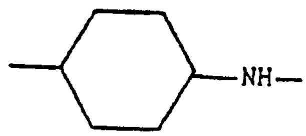

$NH(CH_2)_m$-NH-, $-O(CH_2)_n$-NH- und $-(CH_2)_n$-NH- steht, worin k den Wert 2 oder 3 , m den Wert 2,3,4,5 oder 6 und n den Wert 0,1,2,3 oder 4 haben, und Y für ein Sauerstoffatom oder eine der Gruppen $=NH$, $=N-\underset{\underset{O}{\|}}{C}-R^1$ und $=N-\underset{\underset{O}{\|}}{C}-OR^2$ steht, worin $R^1$ für eine gerad- oder verzweigtkettige $C_1$-$C_6$-Alkylgruppe oder eine gegebenenfalls mit einem oder mehreren Halogenatomen, $C_1$-$C_3$-Alkylgruppen oder $C_1$-$C_3$-Alkoxygruppen substituierte Arylgruppe steht und $R^2$ eine gegebenenfalls mit einem oder mehreren Halogenatomen, $C_1$-$C_3$-Alkoxygruppen oder Phenylresten substituierte, gerad- oder verzweigtkettige $C_1$-$C_4$-Alkylgruppe bedeutet, sowie die physiologisch annehmbaren Salze davon.

2. Diazinderivate nach Anspruch 1, dadurch **gekennzeichnet,** daß A für ein Schwefelatom und B für ein Kohlenstoffatom stehen, R ein Wasserstoffatom, eine $C_1$-$C_3$-Alkylgruppe oder eine Hydroxymethylgruppe und $R'$ ein Wasserstoffatom, eine Nitrogruppe, eine Cyanogruppe oder ein Halogenatom bedeutet, X für eine der Gruppen

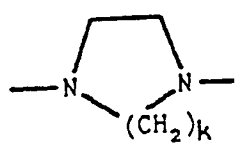

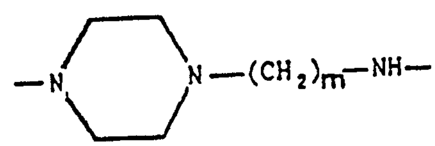

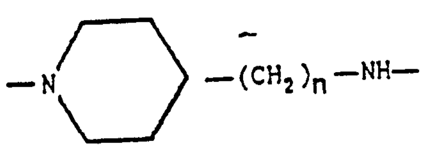

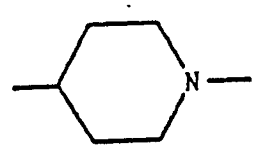

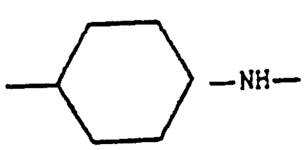

$-NH(CH_2)_m$-NH-, $-O(CH_2)_n$-NH- und $-(CH_2)_n$-NH- steht, worin k den Wert 2 oder 3, m den Wert 2,3,4,5 oder 6 und n den Wert 0,1,2,3 oder 4 haben und Y für die Gruppe $=NH$ steht, sowie die physiologisch annehmbaren Salze davon.

3. Diazinderivate nach Anspruch 1, dadurch **gekennzeichnet,** daß A für ein Stickstoffatom oder die Gruppe $-N-CH_3$ und B für ein Kohlenstoffatom stehen, R ein Wasserstoffatom oder eine $C_1$-$C_3$-Alkylgruppe und $R'$ ein Wasserstoffatom, eine Nitrogruppe, eine Cyanogruppe oder ein Halogenatom bedeutet und Y für

die Gruppe =NH steht, sowie die physiologisch annehmbaren Salze davon.

4. Diazinderivate nach Anspruch 1, dadurch **gekennzeichnet,** daß A für ein Kohlenstoffatom und B für ein Stickstoffatom steht, R ein Wasserstoffatom oder eine Methylgruppe ist und R' ein Wasserstoffatom, eine Nitrogruppe, eine Cyanogruppe oder ein Halogenatom bedeutet und Y für die Gruppe =NH steht, sowie die physiologisch annehmbaren Salze davon.

5. Diazinderivate nach Anspruch 1, dadurch **gekennzeichnet,** daß Y für eine der Gruppen

$=NH$ $=N-\underset{\overset{\|}{O}}{C}-R'$ und $=N-\underset{\overset{\|}{O}}{C}-OR^2$

steht, worin R' für eine gerad- oder verzweigtkettige $C_1$-$C_6$-Alkylgruppe oder eine gegebenenfalls mit einem oder mehreren Halogenatomen, $C_1$-$C_3$-Alkylgruppen oder $C_1$-$C_3$-Alkoxygruppen substituierte Arylgruppe steht und $R^2$ eine gegebenenfalls mit einem oder mehreren Halogenatomen, $C_1$-$C_3$-Alkoxygruppen oder Phenylresten substituierte, gerad- oder verzweigtkettige $C_1$-$C_4$-Alkylgruppe bedeutet, sowie die physiologisch annehmbaren Salze davon.

6. 5-[4-[4-[3-(1H-Imidazol-4-yl)-propylamino-iminomethylen]-piperazin-1-yl]-3-nitro-phenyl]-6-methyl-3H,6H-1,3,4-thiadiazin-2-on und die physiologisch annehmbaren Salze davon.

7. $N^1$-[3-(1H-Imidazol-4-yl)propyl]-$N^3$-[1-[4-(6-methyl-2-oxo-3,6-dihydro-1,3,4-thiadiazin-5-yl)-2-nitrophenyl]-piperidin-4-yl]-guanidin und die physiologisch annehmbaren Salze davon.

8. Verfahren zur Herstellung von Diazinderivaten der allgemeinen Formel I

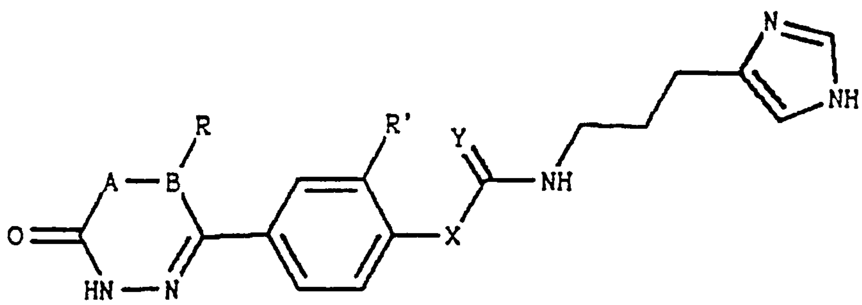

(I)

in der A für ein Schwefelatom und B für ein Kohlenstoffatom steht oder eines der beiden Atome A und B ein unsubstituiertes oder mit einer $C_1$-$C_3$-Alkylgruppe substituiertes Stickstoffatom und das andere ein Kohlenstoffatom bedeuten, R ein Wasserstoffatom, eine $C_1$-$C_3$ Alkylgruppe oder eine Hydroxymethylgruppe und R' ein Wasserstoffatom, eine Nitrogruppe, eine Cyanogruppe oder ein Halogenatom bedeutet, X für eine der Gruppen

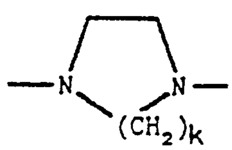

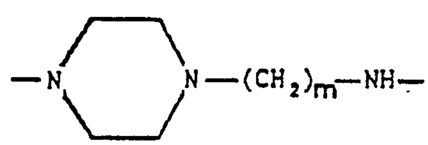

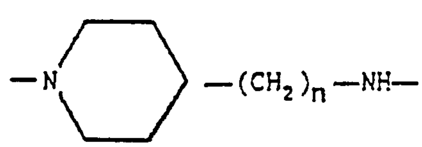

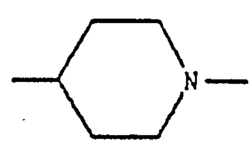

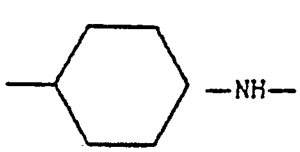

$-NH(CH_2)_m-NH-$, $-O(CH_2)_n-NH-$ und $-(CH_2)_n-NH-$ steht, worin k den Wert 2 oder 3, m den Wert 2,3,4,5 oder 6 und n den Wert 0,1,2,3 oder 4 haben, und Y für ein Sauerstoffatom oder eine der Gruppen

$=NH$ , $=N-\underset{\underset{O}{\|}}{C}-R^1$ und $=N-\underset{\underset{O}{\|}}{C}-OR^2$

steht, worin $R^1$ für eine gerad- oder verzweigtkettige $C_1$-$C_6$-Alkylgruppe oder eine gegebenenfalls mit einem oder mehreren Halogenatomen, $C_1$-$C_3$-Alkylgruppen oder $C_1$-$C_3$-Alkoxygruppen substituierte Arylgruppe steht und $R^2$ eine gegebenenfalls mit einem oder mehreren Halogenatomen, $C_1$-$C_3$-Alkoxygruppen oder Phenylresten substituierte, gerad- oder verzweigtkettige $C_1$-$C_4$-Alkylgruppe bedeutet, sowie der physiologisch annehmbaren Salze davon, dadurch **gekennzeichnet,** daß man

(a) zur Herstellung von Verbindungen der allgemeinen Formel I

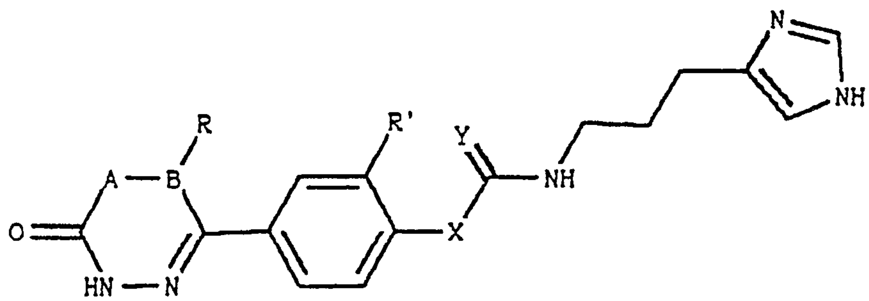

(I)

bei denen A, B, R, R′ und X die obengenannten Bedeutungen besitzen und Y für die Gruppe $=NH$,

$=N-\underset{\underset{O}{\|}}{C}-R^1$ oder $=N-\underset{\underset{O}{\|}}{C}-OR^2$

steht, worin $R^1$ und $R^2$ wie oben definiert sind,

($a_1$) eine Verbindung der allgemeinen Formel II

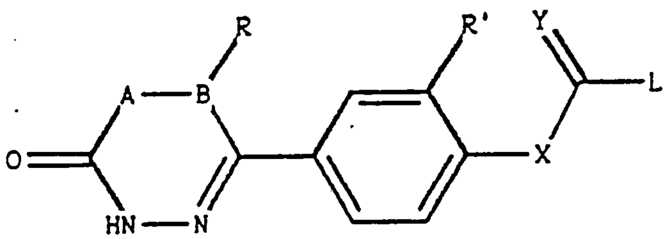

(II)

in der A, B, R, R′, X und Y wie oben definiert sind und L für eine $C_1$-$C_4$-Alkylthio-, eine Phenylthio-, eine $C_1$-$C_4$-Alkoxy- oder eine Phenoxygruppe steht, mit einer Verbindung der Formel III

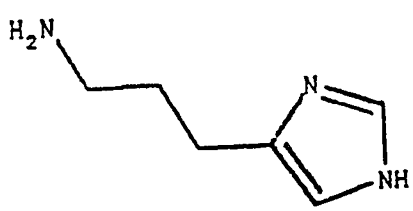

(III)

zu einer Verbindung der allgemeinen Formel I umsetzt oder

($a_2$) eine Verbindung der allgemeinen Formel IV

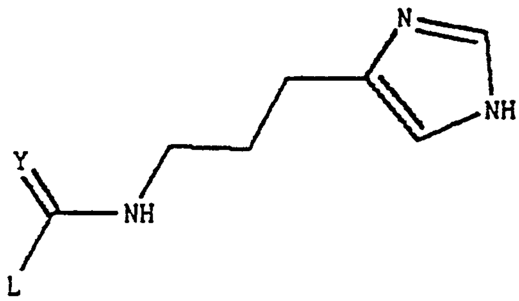

(IV)

in der Y und L die oben unter (a₁) angegebenen Bedeutungen besitzen, mit einer Verbindung der allgemeinen Formel V

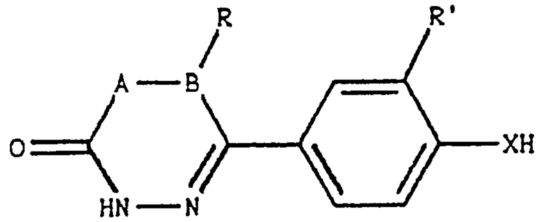

(V)

in der A, B, R, R′ und X wie oben definiert sind, zu einer Verbindung der allgemeinen Formel I umsetzt oder

(b) zur Herstellung von Verbindungen der allgemeinen Formel I, bei denen A, B, R, R′ und X wie oben definiert sind und Y für die Gruppe =NH steht, eine Verbindung der allgemeinen Formel Ia

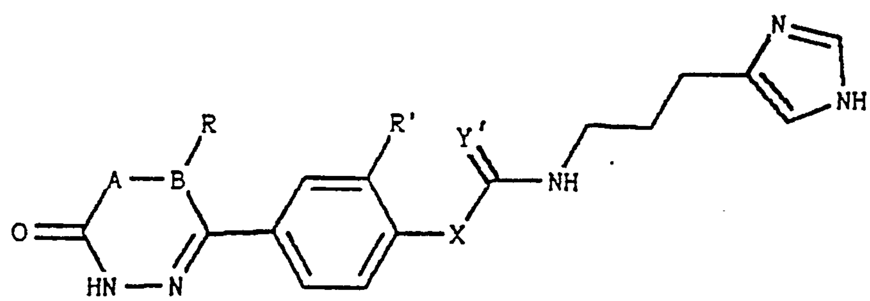

(Ia)

in der A, B, R, R′ und X die oben angegebenen Bedeutungen haben und Y′ für eine der Gruppen $=N-C(=O)-R^1$ und $=N-C(=O)-OR^2$ steht, worin $R^1$ und $R^2$ wie oben definiert sind, sauer oder basisch zu einer Verbindung der allgemeinen Formel I hydrolysiert, oder daß man

(c) zur Herstellung von Verbindungen der allgemeinen Formel I, bei denen A, B, R, R′ und X die oben angegebenen Bedeutungen besitzen und Y für ein Sauerstoffatom steht, eine Verbindung der allgemeinen Formel VI

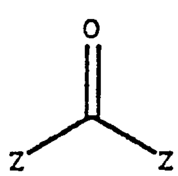

(VI)

in der Z für ein Halogenatom, eine Trichlormethoxygruppe oder den über ein Stickstoffatom gebundenen Rest eines Azols oder Benzazols mit mindestens zwei Stickstoffatomen im Fünfring steht, nacheinander mit

einer Verbindung der allgemeinen Formel V

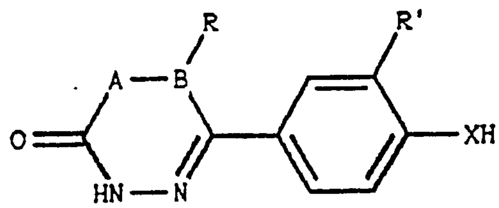

(V)

in der A, B, R, R′ und X wie oben definiert sind, und einer Verbindung der allgemeinen Formel III

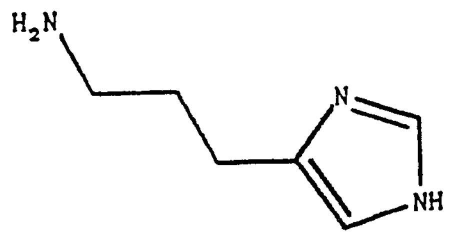

(III)

zu einer Verbindung der allgemeinen Formel I umsetzt und daß man gegebenenfalls die nach den Verfahrensvarianten (a) bis (c) erhaltene Verbindung der allgemeinen Formel I in an sich bekannter Weise in ihr physiologisch annehmbares Salz umwandelt.

9. Arzneimittel, dadurch **gekennzeichnet,** daß es eine Verbindung nach einem der Ansprüche 1 bis 7 zusammen mit mindestens einem inerten, pharmazeutisch annehmbaren Träger oder Verdünnungsmittel enthält.

Patentansprüche für folgenden Vertragsstaat: ES

1. Verfahren zur Herstellung von Diazinderivaten der allgemeinen Formel I

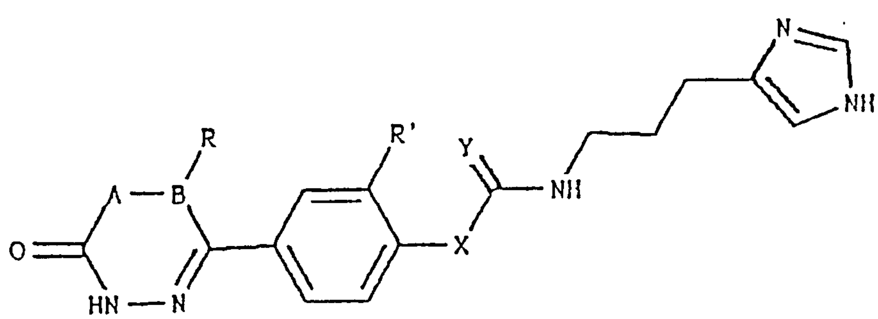

(I)

in der A für ein Schwefelatom und B für ein Kohlenstoffatom steht oder eines der beiden Atome A und B ein unsubstituiertes oder mit einer $C_1$-$C_3$-Alkylgruppe substituiertes Stickstoffatom und das andere ein Kohlenstoffatom bedeuten, R ein Wasserstoffatom, eine $C_1$-$C_3$-Alkylgruppe oder eine Hydroxymethylgruppe und R′ ein Wasserstoffatom, eine Nitrogruppe, eine Cyanogruppe oder ein Halogenatom bedeutet, X für eine der Gruppen

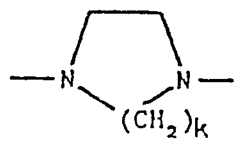

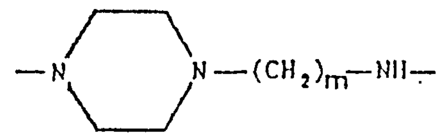

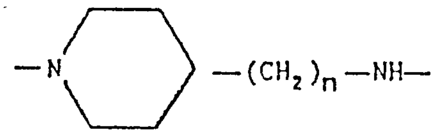

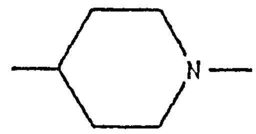

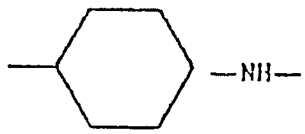

$-NH(CH_2)_m-NH-$, $-O(CH_2)_n-NH-$ und $-(CH_2)_n-NH-$ steht, worin k den Wert 2 oder 3, m den Wert 2,3,4,5 oder 6 und n den Wert 0,1,2,3 oder 4 haben, und Y für ein Sauerstoffatom oder eine der Gruppen $=NH$ $=N-\underset{O}{\underset{\|}{C}}-R'$ und $=N-\underset{O}{\underset{\|}{C}}-OR^2$ steht, worin R' für eine gerad- oder verzweigtkettige $C_1$-$C_6$-Alkylgruppe oder eine gegebenenfalls mit einem oder mehreren Halogenatomen, $C_1$-$C_3$-Alkylgruppen oder $C_1$-$C_3$-Alkoxygruppen substituierte Arylgruppe steht und $R^2$ eine gegebenenfalls mit einem oder mehreren Halogenatomen, $C_1$-$C_3$-Alkoxygruppen oder Phenylresten substituierte, gerad- oder verzweigtkettige $C_1$-$C_4$-Alkylgruppe bedeutet, sowie der physiologisch annehmbaren Salze davon, dadurch **gekennzeichnet,** daß man

(a) zur Herstellung von Verbindungen der allgemeinen Formel I

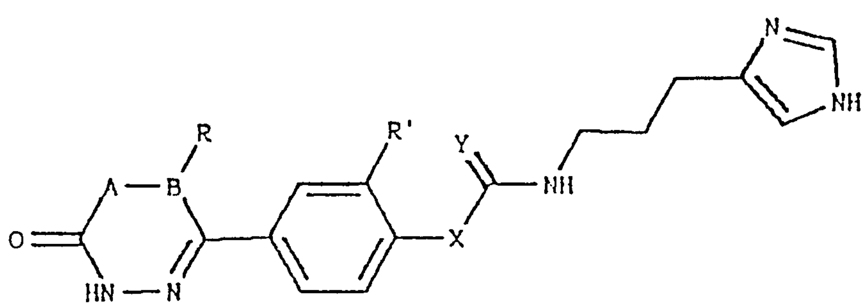

(I)

bei denen A, B, R, R' und X die obengenannten Bedeutungen besitzen und Y für die Gruppe $=NH$, $=N-\underset{O}{\underset{\|}{C}}-R'$ oder $=N-\underset{O}{\underset{\|}{C}}-OR^2$ steht, worin R' und $R^2$ wie oben definiert sind,

($a_1$) eine Verbindung der allgemeinen Formel II

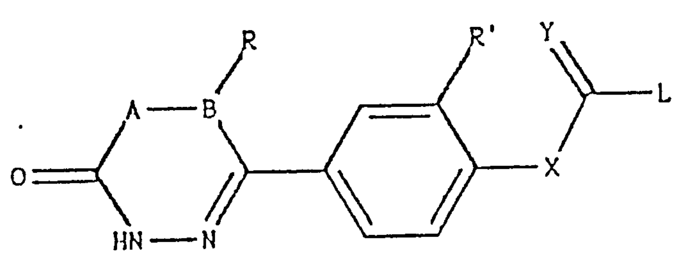

(II)

in der A, B, R, R', X und Y wie oben definiert sind und L für eine $C_1$-$C_4$-Alkylthio-, eine Phenylthio-, eine $C_1$-$C_4$-Alkoxy- oder eine Phenoxygruppe steht, mit einer Verbindung der Formel III

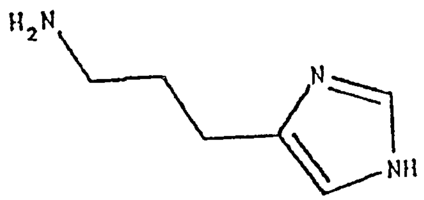

(III)

zu einer Verbindung der allgemeinen Formel I umsetzt oder
($a_2$) eine Verbindung der allgemeinen Formel IV

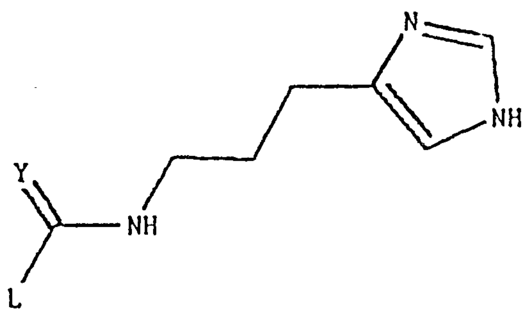

(IV)

in der Y und L die oben unter ($a_1$) angegebenen Bedeutungen besitzen, mit einer Verbindung der allgemeinen Formel V

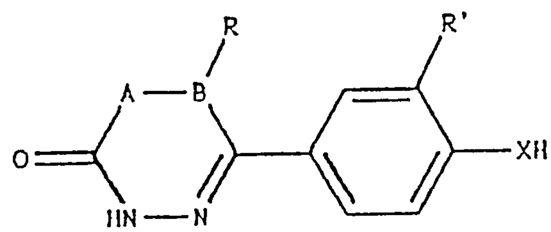

(V)

in der A, B, R, R' und X wie oben definiert sind, zu einer Verbindung der allgemeinen Formel I umsetzt oder
(b) zur Herstellung von Verbindungen der allgemeinen Formel I, bei denen A, B, R, R' und X wie oben definiert sind und Y für die Gruppe =NH steht, eine Verbindung der allgemeinen Formel Ia

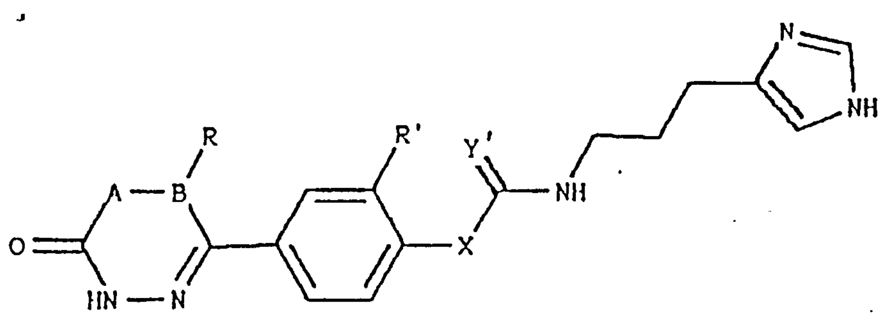

(Ia)

in der A, B, R, R' und X die oben angegebenen Bedeutungen haben und Y' für eine der Gruppen
$=N-\underset{\underset{O}{\parallel}}{C}-R^1$ und $=N-\underset{\underset{O}{\parallel}}{C}-OR^2$

steht, worin $R^1$ und $R^2$ wie oben definiert sind, sauer oder basisch zu einer Verbindung der allgemeinen Formel I hydrolysiert, oder daß man

(c) zur Herstellung von Verbindungen der allgemeinen Formel I, bei denen A, B, R, R′ und X die oben angegebenen Bedeutungen besitzen und Y für ein Sauerstoffatom steht, eine Verbindung der allgemeinen Formel VI

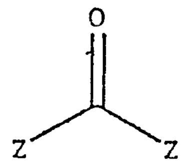

(VI)

in der Z für ein Halogenatom eine Trichlormethoxygruppe oder den über ein Stickstoffatom gebundenen Rest eines Azols oder Benzazols mit mindestens zwei Stickstoffatomen im Fünfring steht, nacheinander mit einer Verbindung der allgemeinen Formel V

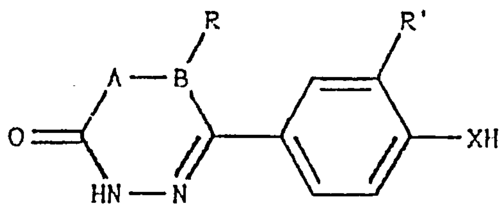

(V)

in der A, B, R, R′ und X wie oben definiert sind, und einer Verbindung der allgemeinen Formel III

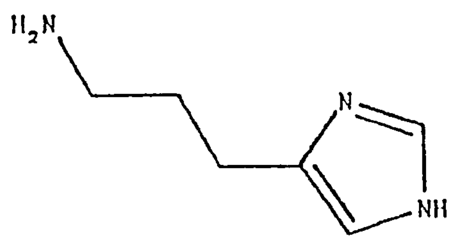

(III)

zu einer Verbindung der allgemeinen Formel I umsetzt und daß man gegebenenfalls die nach den Verfahrensvarianten (a) bis (c) erhaltene Verbindung der allgemeinen Formel I in an sich bekannter Weise in ihr physiologisch annehmbares Salz umwandelt.

2. Verfahren nach Anspruch 1 zur Herstellung von Verbindungen, bei denen A für ein Schwefelatom und B für ein Kohlenstoffatom stehen, R ein Wasserstoffatom, eine $C_1$-$C_3$-Alkylgruppe oder eine Hydroxymethylgruppe und R′ ein Wasserstoffatom, eine Nitrogruppe, eine Cyanogruppe oder ein Halogenatom bedeutet, X für eine der Gruppen

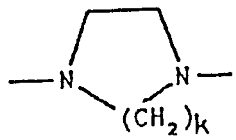

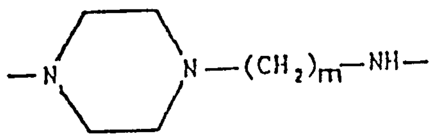

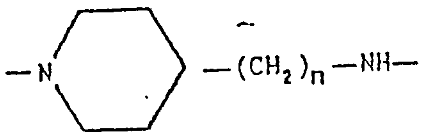

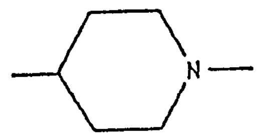

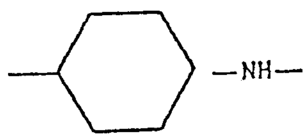

$-NH(CH_2)_m-NH-$, $-O(CH_2)_n-NH-$ und $-(CH_2)_n-NH-$ steht, worin k den Wert 2 oder 3, m den Wert 2,3,4,5 oder 6 und n den Wert 0,1,2,3 oder 4 haben und Y für die Gruppe =NH steht, sowie der physiologisch annehmbaren Salze davon.

3. Verfahren nach Anspruch 1 zur Herstellung von Verbindungen, bei denen A für ein Stickstoffatom oder die Gruppe $-N-CH_3$ und B für ein Kohlenstoffatom stehen, R ein Wasserstoffatom oder eine $C_1$-$C_3$-Alkylgruppe und R′ ein Wasserstoffatom, eine Nitrogruppe, eine Cyanogruppe oder ein Halogenatom bedeutet und Y für die Gruppe =NH steht, sowie der physiologisch annehmbaren Salze davon.

4. Verfahren nach Anspruch 1 zur Herstellung von Verbindungen, bei denen A für ein Kohlenstoffatom und B für ein Stickstoffatom steht, R ein Wasserstoffaton oder eine Methylgruppe ist und R′ ein Wasserstoffatom, eine Nitrogruppe, eine Cyanogruppe oder ein Halogenatom bedeutet und Y für die Gruppe =NH steht, sowie der physiologisch annehmbaren Salze davon.

5. Verfahren nach Anspruch 1 zur Herstellung von Verbindungen, bei denen Y für eine der Gruppen

$=NH$ $=N-\underset{\underset{O}{\|}}{C}-R^1$ und $=N-\underset{\underset{O}{\|}}{C}-OR^2$

steht, worin $R^1$ für eine gerad- oder verzweigtkettige $C_1$-$C_6$-Alkylgruppe oder eine gegebenenfalls mit einem oder mehreren Halogenatomen, $C_1$-$C_3$-Alkylgruppen oder $C_1$-$C_3$-Alkoxygruppen substituierte Arylgruppe steht und $R^2$ eine gegebenenfalls mit einem oder mehreren Halogenatomen, $C_1$-$C_3$-Alkoxygruppen oder Phenylresten substituierte, gerad- oder verzweigtkettige $C_1$-$C_4$ Alkylgruppe bedeutet, sowie der physiologisch annehmbaren Salze davon.

6. Verfahren nach Anspruch 1 zur Herstellung von 5-[4-[4-[3-(1H-Imidazol-4-yl)-propylamino-iminomethylen]-piperazin-1-yl]-3-nitro-phenyl]-6-methyl-3H, 6H-1,3,4-thiadiazin-2-on und der physiologisch annehmbaren Salze davon.

7. Verfahren nach Anspruch 1 zur Herstellung von $N^1$-[3-(1H-Imidazol-4-yl)propyl]-$N^3$-[1-[4-(6-methyl-2-oxo-3,6-dihydro-1,3,4-thiadiazin-5-yl)-2-nitro-phenyl]-piperidin-4-yl]-guanidin und der physiologisch annehmbaren Salze davon.

Patentansprüche für folgenden Vertragsstaat: GR

1. Diazinderivate der allgemeinen Formel I

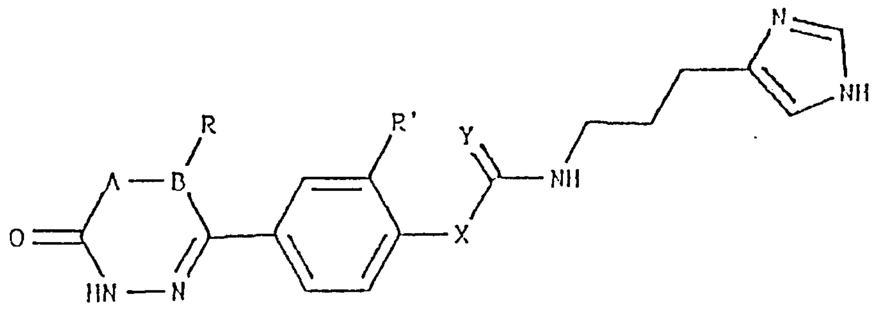

(I)

in der A für ein Schwefelatom und B für ein Kohlenstoffatom steht, oder eines der beiden Atome A und B ein unsubstituiertes oder mit einer $C_1$-$C_3$-Alkylgruppe substituiertes Stickstoffatom und das andere ein Kohlenstoffatom bedeuten, R ein Wasserstoffatom, eine $C_1$-$C_3$-Alkylgruppe oder eine Hydroxymethylgruppe und $R'$ ein Wasserstoffatom, eine Nitrogruppe, eine Cyanogruppe oder ein Halogenatom bedeutet, X für eine der Gruppen

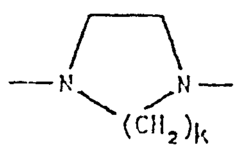

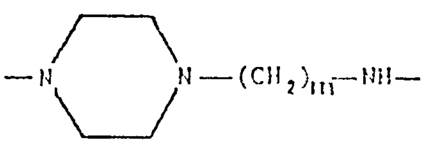

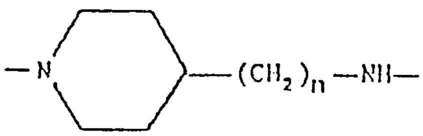

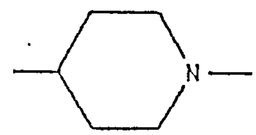

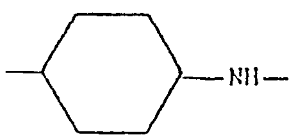

$-NH(CH_2)_m-NH-$, $-O(CH_2)_n-NH-$ und $-(CH_2)_n-NH-$ steht, worin k den Wert 2 oder 3 , m den Wert 2,3,4,5 oder 6 und n den Wert 0,1,2,3 oder 4 haben, und Y für ein Sauerstoffatom oder eine der Gruppen

$=NH$ , $=N-\underset{\underset{O}{\|}}{C}-R^1$ und $=N-\underset{\underset{O}{\|}}{C}-OR^2$

steht, worin $R^1$ für eine gerad- oder verzweigtkettige $C_1$-$C_6$-Alkylgruppe oder eine gegebenenfalls mit einem oder mehreren Halogenatomen, $C_1$-$C_3$-Alkylgruppen oder $C_1$-$C_3$-Alkoxygruppen substituierte Arylgruppe steht und $R^2$ eine gegebenenfalls mit einem oder mehreren Halogenatomen, $C_1$-$C_3$-Alkoxygruppen oder Phenylresten substituierte, gerad- oder verzweigtkettige $C_1$-$C_4$-Alkylgruppe bedeutet, sowie die Salze davon.

2. Diazinderivate nach Anspruch 1, dadurch **gekennzeichnet,** daß A für ein Schwefelatom und B für ein Kohlenstoffatom stehen, R ein Wasserstoffatom, eine $C_1$-$C_3$-Alkylgruppe oder eine Hydroxymethylgruppe und $R'$ ein Wasserstoffatom, eine Nitrogruppe, eine Cyanogruppe oder ein Halogenatom bedeutet, X für eine der Gruppen

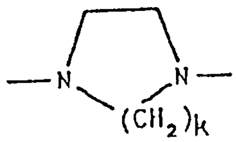

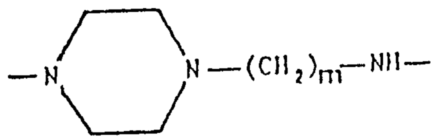

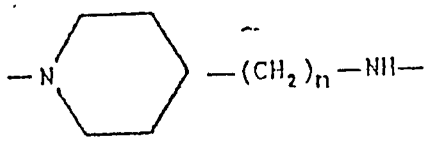

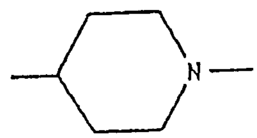

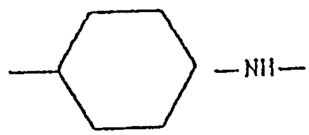

$-NH(CH_2)_m-NH-$, $-O(CH_2)_n-NH-$ und $-(CH_2)_n-NH-$ steht, worin k den Wert 2 oder 3, m den Wert 2,3,4,5 oder 6 und n den Wert 0,1,2,3 oder 4 haben und Y für die Gruppe =NH steht, sowie die Salze davon.

3. Diazinderivate nach Anspruch 1, dadurch **gekennzeichnet,** daß A für ein Stickstoffatom oder die Gruppe $-N-CH_3$ und B für ein Kohlenstoffatom stehen, R ein Wasserstoffaton oder eine $C_1$-$C_3$-Alkylgruppe und R′ ein Wasserstoffatom, eine Nitrogruppe, eine Cyanogruppe oder ein Halogenatom bedeutet und Y für die Gruppe =NH steht, sowie die Salze davon.

4. Diazinderivate nach Anspruch 1, dadurch **gekennzeichnet,** daß A für ein Kohlenstoffatom und B für ein Stickstoffatom steht, R ein Wasserstoffatom oder eine Methylgruppe ist und R′ ein Wasserstoffatom, eine Nitrogruppe, eine Cyanogruppe oder ein Halogenatom bedeutet und Y für die Gruppe =NH steht, sowie die Salze davon.

5. Diazinderivate nach Anspruch 1, dadurch **gekennzeichnet,** daß Y für eine der Gruppen

$=NH$ $=N-\underset{\underset{O}{\|}}{C}-R^1$ und $=N-\underset{\underset{O}{\|}}{C}-OR^2$

steht, worin $R^1$ für eine gerad- oder verzweigtkettige $C_1$-$C_6$-Alkylgruppe oder eine gegebenenfalls mit einem oder mehreren Halogenatomen, $C_1$-$C_3$-Alkylgruppen oder $C_1$-$C_3$-Alkoxygruppen substituierte Arylgruppe steht und $R^2$ eine gegebenenfalls mit einem oder mehreren Halogenatomen, $C_1$-$C_3$-Alkoxygruppen oder Phenylresten substituierte, gerad- oder verzweigtkettige $C_1$-$C_4$-Alkylgruppe bedeutet, sowie die Salze davon.

6. 5-[4-[4-[3-(1H-Imidazol-4-yl)-propylamino-iminomethylen]-piperazin-1-yl]-3-nitro-phenyl]-6-methyl-3H, 6H-1,3,4-thiadiazin-2-on und die Salze davon.

7. $N^1$-[3-(1H-Imidazol-4-yl)propyl]-$N^3$-[1-[4-(6-methyl-2-oxo-3,6-dihydro-1,3,4-thiadiazin-5-yl)-2-nitro-phenyl]-piperidin-4-yl]-guanidin und die Salze davon.

8. Verfahren zur Herstellung von Diazinderivaten der allgemeinen Formel I

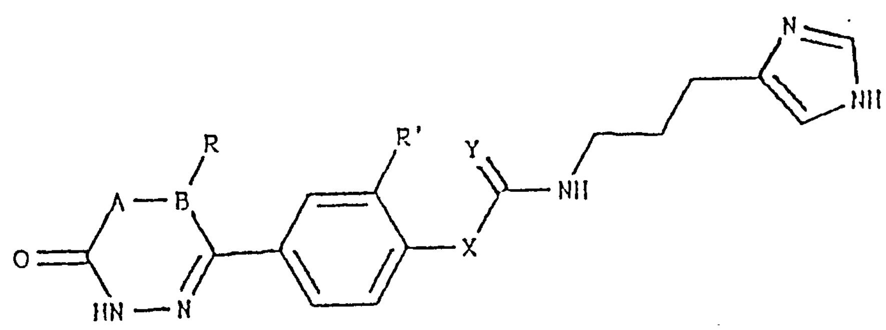

(I)

in der A für ein Schwefelatom und B für ein Kohlenstoffatom steht oder eines der beiden Atome A und B ein unsubstituiertes oder mit einer $C_1$-$C_3$-Alkylgruppe substituiertes Stickstoffatom und das andere ein Kohlenstoffatom bedeuten, R ein Wasserstoffatom, eine $C_1$-$C_3$-Alkylgruppe oder eine Hydroxymethylgruppe

und R' ein Wasserstoffatom, eine Nitrogruppe, eine Cyanogruppe oder ein Halogenatom bedeutet, X für eine der Gruppen

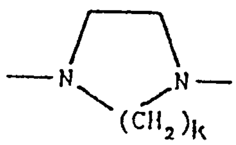

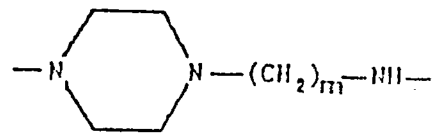

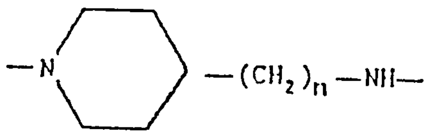

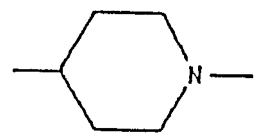

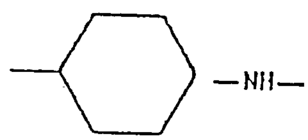

$-NH(CH_2)_m-NH-$, $-O(CH_2)_n-NH-$ und $-(CH_2)_n-NH-$ steht, worin k den Wert 2 oder 3, m den Wert 2,3,4,5 oder 6 und n den Wert 0,1,2,3 oder 4 haben, und Y für ein Sauerstoffatom oder eine der Gruppen $=NH$, $=N-\underset{O}{\underset{\|}{C}}-R^1$ und $=N-\underset{O}{\underset{\|}{C}}-OR^2$ steht worin $R^1$ für eine gerad- oder verzweigtkettige $C_1$-$C_6$-Alkylgruppe oder eine gegebenenfalls mit einem oder mehreren Halogenatomen, $C_1$-$C_3$-Alkylgruppen oder $C_1$-$C_3$-Alkoxygruppen substituierte Arylgruppe steht und $R^2$ eine gegebenenfalls mit einem oder mehreren Halogenatomen, $C_1$-$C_3$-Alkoxygruppen oder Phenylresten substituierte, gerad- oder verzweigtkettige $C_1$-$C_4$-Alkylgruppe bedeutet, sowie der Salze davon, dadurch **gekennzeichnet,** daß man

(a) zur Herstellung von Verbindungen der allgemeinen Formel I

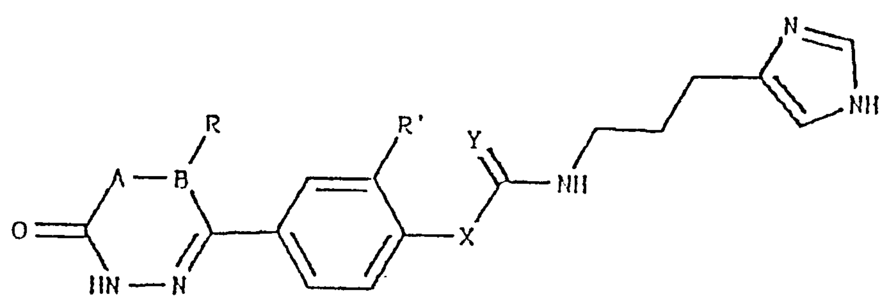

(I)

bei denen A, B, R, R' und X die obengenannten Bedeutungen besitzen und Y für die Gruppe $=NH$, $=N-\underset{O}{\underset{\|}{C}}-R^1$ oder $=N-\underset{O}{\underset{\|}{C}}-OR^2$ steht, worin $R^1$ und $R^2$ wie oben definiert sind,

($a_1$) eine Verbindung der allgemeinen Formel II

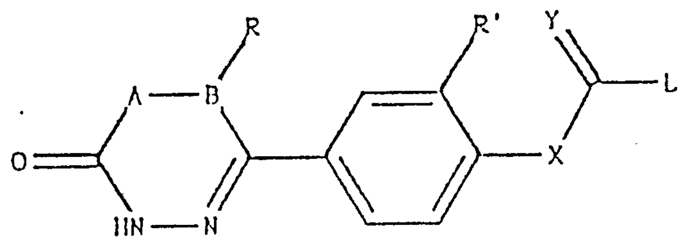

(II)

in der A, B, R, R′, X und Y wie oben definiert sind und L für eine $C_1$-$C_4$-Alkylthio-, eine Phenylthio-, eine $C_1$-$C_4$-Alkoxy- oder eine Phenoxygruppe steht, mit einer Verbindung der Formel III

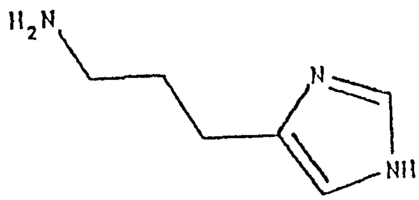

(III)

zu einer Verbindung der allgemeinen Formel I umsetzt oder
($a_2$) eine Verbindung der allgemeinen Formel IV

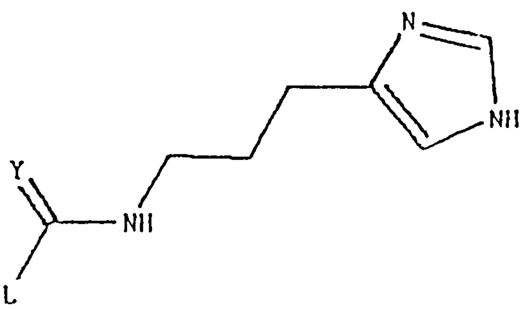

(IV)

in der Y und L die oben unter ($a_1$) angegebenen Bedeutungen besitzen, mit einer Verbindung der allgemeinen Formel V

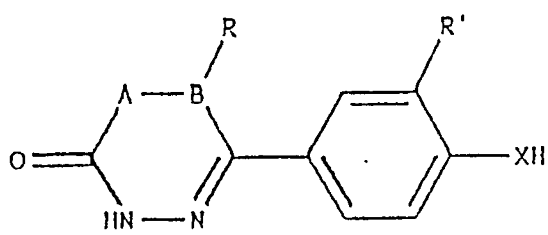

(V)

in der A, B, R, R′ und X wie oben definiert sind, zu einer Verbindung der allgemeinen Formel I umsetzt oder
(b) zur Herstellung von Verbindungen der allgemeinen Formel I, bei denen A, B, R, R′ und X wie oben definiert sind und Y für die Gruppe =NH steht, eine Verbindung der allgemeinen Formel Ia

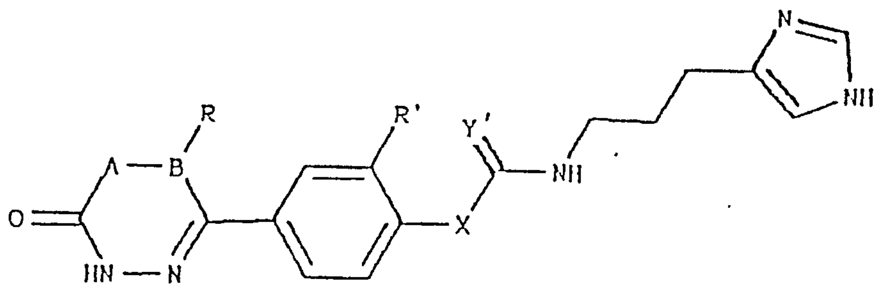

(Ia)

in der A, B, R, R′ und X die oben angegebenen Bedeutungen haben und Y′ für eine der Gruppen $=N-\underset{\underset{O}{\|}}{C}-R'$ und $=N-\underset{\underset{O}{\|}}{C}-OR^2$ steht, worin R' und $R^2$ wie oben definiert sind, sauer oder basisch zu einer Verbindung der allgemeinen Formel I hydrolysiert, oder daß man

(c) zur Herstellung von Verbindungen der allgemeinen Formel I, bei denen A, B, R, R′ und X die oben angegebenen Bedeutungen besitzen und Y für ein Sauerstoffatom steht, eine Verbindung der allgemeinen Formel VI

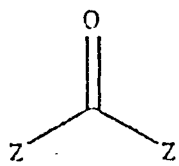

(VI)

in der Z für ein Halogenatom, eine Trichlormethoxygruppe oder den über ein Stickstoffatom gebundenen Rest eines Azols oder Benzazols mit mindestens zwei Stickstoffatomen im Fünfring steht, nacheinander mit einer Verbindung der allgemeinen Formel V

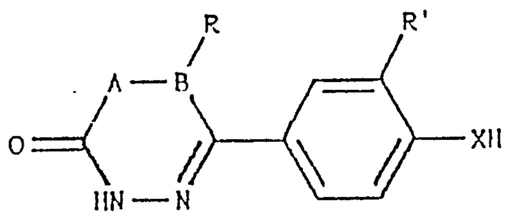

(V)

in der A, B, R, R′ und X wie oben definiert sind, und einer Verbindung der allgemeinen Formel III

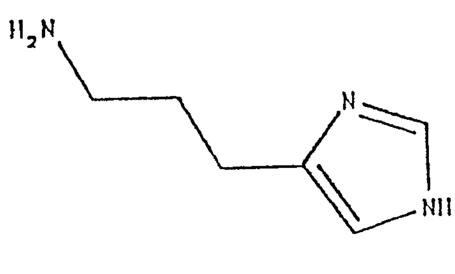

(III)

zu einer Verbindung der allgemeinen Formel I umsetzt und daß man gegebenenfalls die nach den Verfahrensvarianten (a) bis (c) erhaltene Verbindung der allgemeinen Formel I in an sich bekannter Weise in ihr Salz umwandelt.

Europäisches Patentamt

# EUROPÄISCHER RECHERCHENBERICHT

Nummer der Anmeldung

EP 90 10 6877

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 304 534 (HEUMANN PHARMA GMBH UND CO.)<br>* Ansprüche 1,10; Beispiele 6,8,10,11 * | 1,9 | C 07 D 417/12<br>C 07 D 417/14<br>C 07 D 403/12<br>A 61 K 31/54<br>A 61 K 31/53 //<br>(C 07 D 417/12<br>C 07 D 285:00<br>C 07 D 233:00 )<br>(C 07 D 417/14<br>C 07 D 285:00<br>C 07 D 233:00<br>C 07 D 211:00 )<br>(C 07 D 403/12<br>C 07 D 253:00<br>C 07 D 233:00 ) |
| A | * Anspruch 9<br>--- | 8 | |
| D,Y | EP-A-0 052 442 (IMPERIAL CHEMICAL INDUSTRIES PLC)<br>* Ansprüche 1,8,9; Beispiele 11-13,15,23,24,39 *<br>--- | 1,9 | |
| Y | EP-A-0 220 044 (SMITH KLINE AND FRENCH LABORATORIES)<br>* Ansprüche 1,14-16; Beispiel 6,7,10 *<br>--- | 1,9 | |
| Y | EP-A-0 303 418 (LABORATOIRE SOBIO S.A.)<br>* Ansprüche 1,9-12; Beispiele 1-37 *<br>--- | 1,9 | |
| A,P | EP-A-0 339 208 (HEUMANN PHARMA GMBH UND CO.)<br>* Ansprüche 1,2,10,11 *<br>----- | 1,2,8,9 | |

| RECHERCHIERTE SACHGEBIETE (Int. Cl.5) |
|---|
| C 07 D 237/00<br>C 07 D 253/00<br>C 07 D 285/00<br>C 07 D 401/00<br>C 07 D 403/00<br>C 07 D 417/00 |

Der vorliegende Recherchenbericht wurde für alle Patentansprüche erstellt

| Recherchenort | Abschlußdatum der Recherche | Prüfer |
|---|---|---|
| BERLIN | 24-07-1990 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
..............................................................................
& : Mitglied der gleichen Patentfamilie, übereinstimmendes Dokument

EPO FORM 1503 03.82 (P0403)